# EUROPEAN PATENT APPLICATION

(11) **EP 4 269 426 A1**
(43) Date of publication of application: **01.11.2023**
(21) Application number: 22171005.6
(22) Date of filing: 29.04.2022
(51) Int. Cl.: C07K 14/075

(54) **GENE THERAPY**

(71) Applicant: Ospedale San Raffaele S.r.l., 20132 Milano (IT); Universitätsklinikum Hamburg-Eppendorf, 20246 Hamburg (DE)
(72) Inventor: BROCCOLI, Vania, 20132 Milan (IT); GIANNELLI, Serena, 20132 Milan (IT); LUONI, Mirko, 20132 Milan (IT); KÖRBELIN, Jakob, 20246 Hamburg (DE); TREPEL, Martin, 20246 Hamburg (DE)
(74) Representative: D Young & Co LLP

(57) **Abstract**

An adeno-associated viral (AAV) capsid protein, wherein the capsid protein comprises: (a) a peptide comprising or consisting of the amino acid sequence PGVPGRF, or a variant thereof having up to two amino acid substitutions, additions or deletions; or (b) a peptide comprising or consisting of the amino acid sequence NGVRSVG, or a variant thereof having up to two amino acid substitutions, additions or deletions.

## Description

### FIELD OF THE INVENTION

The present invention relates to compounds for use in gene therapy, for example compounds for improving the targeting of cells of the brain, or central and/or peripheral nervous system by a viral or non-viral particle.

### BACKGROUND TO THE INVENTION

Virus-mediated gene-transfer has been considered as a strategy for gene modification in the nervous system, for example for the treatment of diseases of the brain, or central and/or peripheral nervous system.

Gene therapy approaches require vectors (e.g. viral vectors) endowed with both high transduction efficiency and specificity. Moreover, systemic delivery of a gene therapy formulation is generally preferred, however access into the brain and central nervous system (CNS) is limited by the blood-brain barrier (BBB), which restricts the passage of molecules.

Certain AAV vectors have been used in gene therapy, and among all the natural AAV serotypes, AAV9 is known to diffuse into the CNS parenchyma after intravascular delivery. However, its transduction efficiency decreases substantially in adult animals.

An engineered AAV capsid (AAV-PHP.eB) was previously generated and was found to exhibit superior brain transduction in mice, however this behaviour is not conserved in non-human primates (NHPs). Thus, the AAV-PHP.eB has not improved translational potential compared to AAV9 for the treatment of human neurological diseases.

Accordingly, there is a significant need for new approaches to treat diseases which affect the brain or central nervous system.

### SUMMARY OF THE INVENTION

The present inventors have produced targeted modifications in AAV9 capsids that result in enhanced brain transduction capability. In particular, the inventors generated AAV9 capsid libraries and used these for in vivo screening for new variants with enhanced transduction ability through consecutive rounds of selection for the desired tissue. The inventors have surprisingly discovered modifications of capsid proteins that provide for targeting to cells of the brain or central nervous system (e.g. increase the ability to cross the BBB). The modifications also increase the efficiency of transduction of cells of the brain, and decrease tropism for non-brain tissue. Moreover, the resulting capsid proteins have been validated by the inventors in both mice and marmosets.

In one aspect, the invention provides an adeno-associated viral (AAV) capsid protein, wherein the capsid protein comprises a peptide comprising or consisting of the amino acid sequence PGVPGRF (SEQ ID NO: 1), or a variant thereof having up to two amino acid substitutions, additions or deletions.

In one aspect, the invention provides an adeno-associated viral (AAV) capsid protein, wherein the capsid protein comprises a peptide comprising or consisting of the amino acid sequence AGPGVPGRF (SEQ ID NO: 2), or a variant thereof having up to two amino acid substitutions, additions or deletions.

In one aspect, the invention provides an adeno-associated viral (AAV) capsid protein, wherein the capsid protein comprises a peptide comprising or consisting of the amino acid sequence NGVRSVG (SEQ ID NO: 3), or a variant thereof having up to two amino acid substitutions, additions or deletions.

In one aspect, the invention provides an adeno-associated viral (AAV) capsid protein, wherein the capsid protein comprises a peptide comprising or consisting of the amino acid sequence AGNGVRSVG (SEQ ID NO: 4), or a variant thereof having up to two amino acid substitutions, additions or deletions.

In some embodiments, the variant has up to two amino acid substitutions, additions or deletions. In some embodiments, the variant has up to one amino acid substitution, addition or deletion.

In some embodiments, the AAV capsid protein is an AAV VP1 capsid protein.

In some embodiments, the AAV is AAV1, AAV6, AAV6.2, AAV7, AAV9, rh10, rh39 or rh43.

In some embodiments, the AAV is AAV9.

In some embodiments, the peptide is not at the N- or C-terminus of the AAV capsid protein.

In some embodiments, the peptide is positioned immediately after a position corresponding to amino acid 588 of SEQ ID NO: 5.

In some embodiments, the capsid protein comprises or consists of an amino acid sequence that has at least 75%, 80%, 85%, 90%, 95%, 96%, 97%, 98% or 99% sequence identity to SEQ ID NO: 6. In some embodiments, the capsid protein comprises or consists of an amino acid sequence that has at least 75%, 80%, 85%, 90%, 95%, 96%, 97%, 98% or 99% sequence identity to SEQ ID NO: 7.

In some embodiments, the capsid protein comprises or consists of the amino acid sequence of SEQ ID NO: 6. In some embodiments, the capsid protein comprises or consists of the amino acid sequence of SEQ ID NO: 7.

In some embodiments, the capsid protein comprises or consists of an amino acid sequence that has at least 75%, 80%, 85%, 90%, 95%, 96%, 97%, 98% or 99% sequence identity to SEQ ID NO: 8. In some embodiments, the capsid protein comprises or consists of an amino acid sequence that has at least 75%, 80%, 85%, 90%, 95%, 96%, 97%, 98% or 99% sequence identity to SEQ ID NO: 9.

In some embodiments, the capsid protein comprises or consists of the amino acid sequence of SEQ ID NO: 8. In some embodiments, the capsid protein comprises or consists of the amino acid sequence of SEQ ID NO: 9.

In some embodiments, the capsid protein further comprises the mutation A587G, wherein the amino acids are numbered with reference to SEQ ID NO: 5. In some embodiments, the capsid protein further comprises the mutation Q592A, wherein the amino acids are numbered with reference to SEQ ID NO: 5. In some embodiments, the capsid protein further comprises the mutations A587G and Q592A, wherein the amino acids are numbered with reference to SEQ ID NO: 5.

In some embodiments, the capsid protein further comprises the mutation W503A, wherein the amino acids are numbered with reference to SEQ ID NO: 5.

In some embodiments, the capsid protein further comprises the mutations A587G and W503A, wherein the amino acids are numbered with reference to SEQ ID NO: 5. In some embodiments, the capsid protein further comprises the mutations Q592A and W503A, wherein the amino acids are numbered with reference to SEQ ID NO: 5. In some embodiments, the capsid protein further comprises the mutations A587G, Q592A and W503A, wherein the amino acids are numbered with reference to SEQ ID NO: 5.

In some embodiments, the capsid protein further comprises the mutation K449R, wherein the amino acids are numbered with reference to SEQ ID NO: 5.

In some embodiments, the capsid protein further comprises the mutations A587G, Q592A, W503A and K449R, wherein the amino acids are numbered with reference to SEQ ID NO: 5. In some embodiments, the capsid protein comprises or consists of an amino acid sequence that has at least 75%, 80%, 85%, 90%, 95%, 96%, 97%, 98% or 99% sequence identity to SEQ ID NO: 10.

In some embodiments, the capsid protein comprises or consists of the amino acid sequence of SEQ ID NO: 10.

In some embodiments, the capsid protein comprises or consists of an amino acid sequence that has at least 75%, 80%, 85%, 90%, 95%, 96%, 97%, 98% or 99% sequence identity to SEQ ID NO: 11.

In some embodiments, the capsid protein comprises or consists of the amino acid sequence of SEQ ID NO: 11.

In one aspect, the invention provides an adeno-associated viral (AAV) particle comprising the AAV capsid protein of the invention.

In some embodiments, the AAV particle comprises a polynucleotide comprising a nucleotide sequence of interest.

In some embodiments, the polynucleotide further comprises one or more AAV ITR. In some embodiments, the polynucleotide further comprises two AAV ITRs. In some embodiments, the polynucleotide further comprises a 5' AAV ITR. In some embodiments, the polynucleotide further comprises a 3' AAV ITR. In some embodiments, the polynucleotide further comprises a 5' AAV ITR and a 3' AAV ITR.

In some embodiments, the AAV ITR is an AAV9 ITR.

In some embodiments, the AAV ITR is an AAV2 ITR.

In some embodiments, the nucleotide sequence of interest is operably linked to a promoter. In some embodiments, the nucleotide sequence of interest is operably linked to an enhancer.

In one aspect, the invention provides a pharmaceutical composition comprising the adeno-associated viral (AAV) particle of the invention, and a pharmaceutically acceptable carrier, diluent or excipient.

In one aspect, the invention provides the adeno-associated viral (AAV) particle of the invention for use in therapy.

In one aspect, the invention provides use of the adeno-associated viral (AAV) particle of the invention for the manufacture of a medicament.

In one aspect, the invention provides a method of treating or preventing a disease, the method comprising a step of administering the adeno-associated viral (AAV) particle of the invention to a subject in need thereof.

In one aspect, the invention provides the adeno-associated viral (AAV) particle of the invention for use in treating or preventing a disease of the brain or central nervous system.

In one aspect, the invention provides use of the adeno-associated viral (AAV) particle of the invention for the manufacture of a medicament for treating or preventing a disease of the brain or central nervous system.

In one aspect, the invention provides a method of treating or preventing a disease of the brain or central nervous system, the method comprising a step of administering the adeno-associated viral (AAV) particle of the invention to a subject in need thereof.

In one aspect, the invention provides the adeno-associated viral (AAV) particle of the invention for use in treating or preventing a disease of the peripheral nervous system.

In one aspect, the invention provides use of the adeno-associated viral (AAV) particle of the invention for the manufacture of a medicament for treating or preventing a disease of the peripheral nervous system.

In one aspect, the invention provides a method of treating or preventing a disease of the peripheral nervous system, the method comprising a step of administering the adeno-associated viral (AAV) particle of the invention to a subject in need thereof.

In some embodiments, the disease is a neurological disease.

In some embodiments, the disease is a neurodegenerative disease.

In some embodiments, the disease is dementia.

In some embodiments, the disease is selected from the group consisting of Parkinson's disease, Parkinson's dementia, Lewy Body dementia, diffuse Lewy body dementia, Lewy body variant of Alzheimer's disease, multiple system atrophy and Gaucher disease.

In some embodiments, the AAV particle is administered systemically. In some embodiments, the AAV particle is administered locally in the brain.

In some embodiments, the AAV particle is administered intravenously. In some embodiments, the AAV particle is administered intrathecally.

In some embodiments, the subject is a human.

In some embodiments, the subject is an adult.

In some embodiments, the subject is a juvenile.

In some embodiments, the subject is a paediatric subject. In some embodiments, the subject is a neonatal subject or an infantile subject.

In one aspect, the invention provides use of a peptide for targeting a viral particle to cells of the brain or central nervous system, wherein the peptide:
(a) comprises or consists of the amino acid sequence PGVPGRF (SEQ ID NO: 1), or a variant thereof having up to two amino acid substitutions, additions or deletions; or
(b) comprises or consists of the amino acid sequence NGVRSVG (SEQ ID NO: 3), or a variant thereof having up to two amino acid substitutions, additions or deletions.

In one aspect, the invention provides use of a peptide for targeting a viral particle to cells of the brain or central nervous system, wherein the peptide:
(a) comprises or consists of the amino acid sequence AGPGVPGRF (SEQ ID NO: 2), or a variant thereof having up to two amino acid substitutions, additions or deletions; or
(b) comprises or consists of the amino acid sequence AGNGVRSVG (SEQ ID NO: 4), or a variant thereof having up to two amino acid substitutions, additions or deletions.

In one aspect, the invention provides use of an adeno-associated viral (AAV) capsid protein of the invention for targeting a viral particle to cells of the brain or central nervous system.

In one aspect, the invention provides use of a peptide for targeting a viral particle to cells of the peripheral nervous system, wherein the peptide:
(a) comprises or consists of the amino acid sequence PGVPGRF (SEQ ID NO: 1), or a variant thereof having up to two amino acid substitutions, additions or deletions; or
(b) comprises or consists of the amino acid sequence NGVRSVG (SEQ ID NO: 3), or a variant thereof having up to two amino acid substitutions, additions or deletions.

In one aspect, the invention provides use of a peptide for targeting a viral particle to cells of the peripheral nervous system, wherein the peptide:
(a) comprises or consists of the amino acid sequence AGPGVPGRF (SEQ ID NO: 2), or a variant thereof having up to two amino acid substitutions, additions or deletions; or
(b) comprises or consists of the amino acid sequence AGNGVRSVG (SEQ ID NO: 4), or a variant thereof having up to two amino acid substitutions, additions or deletions.

In one aspect, the invention provides use of an adeno-associated viral (AAV) capsid protein of the invention for targeting a viral particle to cells of the peripheral nervous system.

In one aspect, the invention provides use of a peptide for increasing the efficiency of transduction of cells of the brain by a viral particle, wherein the peptide:
(a) comprises or consists of the amino acid sequence PGVPGRF (SEQ ID NO: 1), or a variant thereof having up to two amino acid substitutions, additions or deletions; or
(b) comprises or consists of the amino acid sequence NGVRSVG (SEQ ID NO: 3), or a variant thereof having up to two amino acid substitutions, additions or deletions.

In one aspect, the invention provides use of a peptide for increasing the efficiency of transduction of cells of the brain by a viral particle, wherein the peptide:
(a) comprises or consists of the amino acid sequence AGPGVPGRF (SEQ ID NO: 2), or a variant thereof having up to two amino acid substitutions, additions or deletions; or
(b) comprises or consists of the amino acid sequence AGNGVRSVG (SEQ ID NO: 4), or a variant thereof having up to two amino acid substitutions, additions or deletions.

In one aspect, the invention provides use of an adeno-associated viral (AAV) capsid protein of the invention for increasing the efficiency of transduction of cells of the brain by a viral particle.

In one aspect, the invention provides use of a peptide for decreasing tropism of a viral particle for non-brain tissue, wherein the peptide:
(a) comprises or consists of the amino acid sequence PGVPGRF (SEQ ID NO: 1), or a variant thereof having up to two amino acid substitutions, additions or deletions; or
(b) comprises or consists of the amino acid sequence NGVRSVG (SEQ ID NO: 3), or a variant thereof having up to two amino acid substitutions, additions or deletions.

In one aspect, the invention provides use of a peptide for decreasing tropism of a viral particle for non-brain tissue, wherein the peptide:
(a) comprises or consists of the amino acid sequence AGPGVPGRF (SEQ ID NO: 2), or a variant thereof having up to two amino acid substitutions, additions or deletions; or
(b) comprises or consists of the amino acid sequence AGNGVRSVG (SEQ ID NO: 4), or a variant thereof having up to two amino acid substitutions, additions or deletions.

In one aspect, the invention provides use of an adeno-associated viral (AAV) capsid protein of the invention for decreasing tropism of a viral particle for non-brain tissue.

In some embodiments, the viral particle is an AAV particle.

In some embodiments, the non-viral particle is a nanoparticle. In some embodiments, the non-viral particle is a lipid nanoparticle.

In some embodiments, the cells are cells of the cerebral cortex, caudate nucleus, thalamus and/or cerebellum.

In some embodiments, the cells are brain endothelial cells, brain astrocytes or brain neuronal cells. In some embodiments, the cells are brain endothelial cells. In some embodiments, the cells are brain astrocytes. In some embodiments, the cells are brain neuronal cells.

In some embodiments, the non-brain tissue is liver, spleen, lung, kidney, heart and/or muscle. In some embodiments, the non-brain tissue is liver.

In one aspect, the invention provides a polynucleotide comprising or consisting of a nucleotide sequence that has at least 75%, 80%, 85%, 90%, 95%, 96%, 97%, 98% or 99% sequence identity to SEQ ID NO: 12.

In one aspect, the invention provides a polynucleotide comprising or consisting of the nucleotide sequence of SEQ ID NO: 12.

In one aspect, the invention provides a polynucleotide comprising or consisting of a nucleotide sequence that has at least 75%, 80%, 85%, 90%, 95%, 96%, 97%, 98% or 99% sequence identity to SEQ ID NO: 13.

In one aspect, the invention provides a polynucleotide comprising or consisting of the nucleotide sequence of SEQ ID NO: 13.

In one aspect, the invention provides a cell comprising a polynucleotide of the invention. In some embodiments, the cell further comprises a polynucleotide comprising a nucleotide sequence of interest and/or a helper plasmid. In some embodiments, the cell is a HEK293 cell or a HEK293T cell.

In one aspect, the invention provides a method for producing an adeno-associated viral (AAV) particle comprising expressing a polynucleotide of the invention in a cell. In some embodiments, the cell further comprises a polynucleotide comprising a nucleotide sequence of interest and/or a helper plasmid. In some embodiments, the cell is a HEK293 cell or a HEK293T cell.

### DESCRIPTION OF THE DRAWINGS

**FIGURE 1**
   Design of the DNA viral vector library with a random 7 amino acid sequence insertion between position 588-589 of the AAV9-VP1 capsid protein. Illustration of the principal step for each round of the in vivo selection for modified viral capsids able to penetrate the brain tissue after administration of the AAV capsid library intravenously through tail vein injection in adult BALB/c mice.
**FIGURE 2**
   Pie-charts representing the relative fraction of sequences relative to each different AAV-capsid in the different organs analyzed in the final round 4 of the viral selection. AAV-Se2 is the most represented sequence on the next-generation sequencing output from the brain lysates. Conversely, AAV-Se2 sequence is not highly enriched in peripheral organs like liver, kidney, heart, muscles and lung.
**FIGURE 3**
   Illustration of the 7 amino acid peptides and the flanking regions specific for AAV-Se1 and AAV-Se2 which are significantly different from those of other AAV capsids such as AAV-PHP.B, AAV-PHP.eB, AAV-PHP.S and AAV-PHP.V1.
**FIGURE 4**
   Immunofluorescence stainings for the viral reporter ZsGreen and markers specific for neurons (NeuN) and endothelium (CD31) on sections of cerebral cortex of mice transduced with AAV-Se2 and AAV9 at the same viral dose (1 × 10(11) vg/animal). Quantifications of the infected neurons or endothelial cells show a marked transduction of AAV-Se2 of brain endothelium, but not neurons, compared to AAV9.
**FIGURE 5**
   Immunofluorescence stainings for the viral reporter ZsGreen and the astrocytic specific marker Sox9 on sections of cerebral cortex of mice transduced with AAV-Se2 and AAV9 at the same viral dose (1 × 10(11) vg/animal). Quantifications of the infected astrocytes show a marked transduction of AAV-Se2 in brain tissues compared to AAV9.
**FIGURE 6**
   Graphs illustrating the viral DNA copies quantified by qPCR on lysates of brain and liver transduced with AAV9 or AAV-Se2 in C57BI/6 and BALB/C strains. A significant higher number of viral AAV-Se2 DNA copies are detected in brain tissues respect to AAV9. Conversely, liver lysates showed an increased number of AAV9 DNA copies respect to AAV-Se2.
**FIGURE 7**
   Immunofluorescence staining for the viral reporter GFP and the endothelium marker CD31 on sections of the cerebral cortex of mice transduced with the AAV-Se1-A503 capsid. GFP expression is mainly confined to brain endothelial cells with an efficiency of transduction of 80% of the whole endothelial cell population in the cerebral cortex and hippocampus. CC, cerebral cortex; HIP, hippocampus.
**FIGURE 8**
   Immunofluorescence staining with the viral reporter GFP and the neuronal marker NeuN in brain sections of mice transduced at newborn stage (P2) and analyzed 4 weeks later. GFP/NeuN double staining shows that between 40% to 55% of neurons in the cerebral cortex are productively transduced with AAV-Se2.
**FIGURE 9**
   Illustration of the procedure to transduce adult marmosets through AAV-Se2 or AAV9 injections into the femoral vein at the dose of 1 × 10(13) vg per animal. 3 weeks after viral delivery the animals are euthanized for tissue inspection and viral transduction analysis.
**FIGURE 10**
   Representative images of immunohistochemistry for the reporter ZsGreen expressed by the AAV2-Se2 in a marmoset brain section (A) and in selected brain regions such as cerebral cortex (B, C), caudate nucleus (D) and thalamus (E).
**FIGURE 11**
   Double immunofluorescence stainings for the viral reporter ZsGreen and specific markers of neurons (NeuN) and astrocytes (GFAP) on cerebral cortex sections of marmosets transduced with AAV-Se2 capsid. Graphs show the efficiency of transduction of AAV-Se2 in neurons and astrocytes in cerebral cortex, caudate nucleus and thalamus.
**FIGURE 12**
   Viral DNA copies quantified by qPCRs on lysates of brain tissues, liver heart and muscles of the 2 marmosets transduced with either AAV-Se2 or standard AAV9.

### DETAILED DESCRIPTION OF THE INVENTION

The terms "comprising", "comprises" and "comprised of" as used herein are synonymous with "including" or "includes"; or "containing" or "contains", and are inclusive or open-ended and do not exclude additional, non-recited members, elements or steps. The terms "comprising", "comprises" and "comprised of" also include the term "consisting of".

### BLOOD-BRAIN BARRIER (BBB)

The term "blood brain barrier" (BBB) may refer to the highly selective semi-permeable membrane barrier which separates the circulating blood from the brain and extracellular fluid in the central nervous system. It is formed by the selectivity of tight junctions between endothelial cells. The blood-brain barrier occurs along all capillaries of the brain and consists of tight junctions.

Overcoming the difficulty of delivering therapeutic agents to specific regions of the brain presents a major challenge to treatment of most brain disorders.

In one aspect, the invention provides an AAV vector particle adapted for crossing the blood-brain barrier.

Preferably, the AAV vector particle is adapted for crossing an intact blood brain barrier. Preferably the AAV vector particle does not impair blood-brain barrier integrity and/or selectivity and/or affect permeability.

In other words, the vector particle is adapted to cross a blood-brain barrier which has not been compromised or weakened, i.e. which maintains tight junctions between endothelial cells. Methods are known in the art that can determine whether or not a blood brain barrier is intact. For example, the permeability of the blood-brain barrier can be detected by perfusion of Evan's blue dye. Alternatively a fluorescent-conjugated cadaverine dye can be used as a blood-brain barrier permeability marker, together with the AAV particle carrying a fluorescent marker.

Suitably, the AAV vector particle of the invention does not cause microgliosis. Suitably, the AAV vector particle does not cause sustained inflammation in the central nervous system.

The "central nervous system" as used herein may refer to the nervous system consisting of the brain and spinal cord.

The "peripheral nervous system" as used herein may refer to the components of the nervous system outside of the central nervous system. The peripheral nervous system consists of the nerves and ganglia outside of the brain and spinal cord.

### Brain endothelium or brain microvascular endothelial cells

As used herein "brain endothelial cells", "brain endothelium" or "brain microvascular endothelial cells" may refer to the cerebral endothelial cells that present the interface between the blood and the central nervous system, i.e. blood-brain barrier endothelial cells. Suitably the brain endothelial cells may form the blood-brain barrier.

In some embodiments, the AAV vector particle targets the brain endothelium. In some embodiments, the AAV vector particle targets the endothelium in at least one of the cerebral cortex, caudate nucleus, thalamus and cerebellum. Preferably, the AAV vector particle targets the endothelium in the cerebral cortex, caudate nucleus, thalamus and cerebellum.

In some embodiments, the AAV vector particle targets the blood-brain barrier-associated endothelial cells. In some embodiments, the AAV vector particle targets the blood-brain barrier associated endothelium of the entire central nervous system, such as including the spinal cord.

As used herein, the term "targets" or "targeting" may mean that the concentration of the AAV vector particle is increased in a specific cell type relative to other cell types. For example, after administration the AAV vector particle may be found in higher concentrations in endothelial cells compared to other cell types, for example pericytes.

Methods for assessing the targeting of the viral vector particle will be known to the skill ed person. For example, to confirm endothelial targeting, one may use immunohistochemistry. A viral vector particle comprising a fluorescent tag could be used along with antibodies against an endothelial cell marker (such as CD31) and a cell marker for another cell type, such as CD13 which is a marker for pericytes. The viral vector particle will demonstrate increased co-localisation with the fluorescence marker of the targeted cell type.

As used herein "peripheral organs" include but are not limited to the liver, spleen, lung, kidney, heart and muscle.

In some embodiments, the AAV vector particle does not substantially target the peripheral organs. Preferably, the AAV vector particle crosses the blood-brain barrier and does not substantially target the peripheral organs. Preferably, the AAV vector particle targets blood-brain endothelial cells and does not substantially target the peripheral organs. Suitably, the peripheral organs do not comprise the AAV vector particle.

### VECTORS

A vector is a tool that allows or facilitates the transfer of an entity from one environment to another. In accordance with the invention, and by way of example, some vectors used in recombinant nucleic acid techniques allow entities, such as a segment of nucleic acid (e.g. a heterologous DNA segment, such as a heterologous cDNA segment), to be transferred into a target cell. The vector may serve the purpose of maintaining the heterologous nucleic acid (DNA or RNA) within the cell, facilitating the replication of the vector comprising a segment of nucleic acid and/or facilitating the expression of the protein encoded by a segment of nucleic acid.

Vectors may include a promoter for the expression of a polynucleotide and optionally a regulator of the promoter.

Vectors comprising polynucleotides may be introduced into cells using a variety of techniques known in the art, such as transfection, transduction and transformation.

Transfection may refer to a general process of incorporating a nucleic acid into a cell and includes a process using a non-viral vector to deliver a polynucleotide to a cell. Transduction may refer to a process of incorporating a nucleic acid into a cell using a viral vector.

The viral particle of the invention may be a viral vector, for example may comprise a polynucleotide comprising a nucleotide sequence of interest.

### ADENO-ASSOCIATED VIRUS (AAV)

In one aspect, the invention provides an adeno-associated viral (AAV) particle comprising the AAV capsid protein of the invention.

Methods of preparing and modifying viral vectors and viral vector particles, such as those derived from AAV, are well known in the art.

The AAV particle may comprise an AAV genome or a fragment or derivative thereof.

An AAV genome is a polynucleotide sequence, which may encode functions needed for production of an AAV particle. These functions include those operating in the replication and packaging cycle of AAV in a host cell, including encapsidation of the AAV genome into an AAV particle. Naturally occurring AAVs are replication-deficient and rely on the provision of helper functions in trans for completion of a replication and packaging cycle. Accordingly, the AAV genome of the AAV vector of the invention is typically replication-deficient.

The AAV genome may be in single-stranded form, either positive or negative-sense, or alternatively in double-stranded form. The use of a double-stranded form allows bypass of the DNA replication step in the target cell and so can accelerate transgene expression.

The AAV genome may be from any naturally derived serotype, isolate or clade of AAV. Thus, the AAV genome may be the full genome of a naturally occurring AAV. As is known to the skilled person, AAVs occurring in nature may be classified according to various biological systems.

Commonly, AAVs are referred to in terms of their serotype. A serotype corresponds to a variant subspecies of AAV which, owing to its profile of expression of capsid surface antigens, has a distinctive reactivity which can be used to distinguish it from other variant subspecies. Typically, a virus having a particular AAV serotype does not efficiently cross-react with neutralising antibodies specific for any other AAV serotype.

AAV serotypes include AAV1, AAV2, AAV3, AAV4, AAV5, AAV6, AAV7, AAV8, AAV9, AAV10 and AAV11, and also recombinant serotypes, such as Rec2 and Rec3, recently identified from primate brain.

In some embodiments, the AAV is AAV1, AAV6, AAV6.2, AAV7, AAV9, rh10, rh39 or rh43. In some embodiments, the AAV capsid protein is derived from an AAV1, AAV6, AAV6.2, AAV7, AAV9, rh10, rh39 or rh43 capsid protein.

Reviews of AAV serotypes may be found in Choi et al. (2005) Curr. Gene Ther. 5: 299-310 and Wu et al. (2006) Molecular Therapy 14: 316-27. The sequences of AAV genomes or of elements of AAV genomes including ITR sequences, rep or cap genes may be derived from the following accession numbers for AAV whole genome sequences: Adeno-associated virus 1 NC_002077, AF063497; Adeno-associated virus 2 NC_001401; Adeno-associated virus 3 NC_001729; Adeno-associated virus 3B NC_001863; Adeno-associated virus 4 NC_001829; Adeno-associated virus 5 Y18065, AF085716; Adeno-associated virus 6 NC_001862; Avian AAV ATCC VR-865 AY186198, AY629583, NC_004828; Avian AAV strain DA-1 NC_006263, AY629583; Bovine AAV NC_005889, AY388617.

### AAV capsid protein

In one aspect, the invention provides an adeno-associated viral (AAV) capsid protein, wherein the capsid protein comprises a peptide comprising or consisting of the amino acid sequence PGVPGRF (SEQ ID NO: 1), or a variant thereof having up to two amino acid substitutions, additions or deletions. In one aspect, the invention provides an adeno-associated viral (AAV) capsid protein, wherein the capsid protein comprises a peptide comprising or consisting of the amino acid sequence AGPGVPGRF (SEQ ID NO: 2), or a variant thereof having up to two amino acid substitutions, additions or deletions.

In one aspect, the invention provides an adeno-associated viral (AAV) capsid protein, wherein the capsid protein comprises a peptide comprising or consisting of the amino acid sequence NGVRSVG (SEQ ID NO: 3), or a variant thereof having up to two amino acid substitutions, additions or deletions. In one aspect, the invention provides an adeno-associated viral (AAV) capsid protein, wherein the capsid protein comprises a peptide comprising or consisting of the amino acid sequence AGNGVRSVG (SEQ ID NO: 4), or a variant thereof having up to two amino acid substitutions, additions or deletions.

In some embodiments, the AAV capsid protein is an AAV VP1 capsid protein.

In some embodiments, the AAV is AAV1, AAV6, AAV6.2, AAV7, AAV9, rh10, rh39 or rh43.

In some embodiments, the AAV is AAV9.

An example wild type AAV9 capsid protein amino acid sequence is: 
MAADGYLPDWLEDNLSEGIREWWALKPGAPQPKANQQHQDNARGLVLPGYKYLGPGNGLDKG
EPVNAADAAALEHDKAYDQQLKAGDNPYLKYNHADAEFQERLKEDTSFGGNLGRAVFQAKKR
LLEPLGLVEEAAKTAPGKKRPVEQSPQEPDSSAGIGKSGAQPAKKRLNFGQTGDTESVPDPQ
PIGEPPAAPSGVGSLTMASGGGAPVADNNEGADGVGSSSGNWHCDSQWLGDRVITTSTRTWA
LPTYNNHLYKQISNSTSGGSSNDNAYFGYSTPWGYFDFNRFHCHFSPRDWQRLINNNWGFRP
KRLNFKLFNIQVKEVTDNNGVKTIANNLTSTVQVFTDSDYQLPYVLGSAHEGCLPPFPADVF
MIPQYGYLTLNDGSQAVGRSSFYCLEYFPSQMLRTGNNFQFSYEFENVPFHSSYAHSQSLDR
LMNPLIDQYLYYLSKTINGSGQNQQTLKFSVAGPSNMAVQGRNYIPGPSYRQQRVSTTVTQN
NNSEFAWPGASSWALNGRNSLMNPGPAMASHKEGEDRFFPLSGSLIFGKQGTGRDNVDADKV
 
MITNEEEIKTTNPVATESYGQVATNHQSAQAQAQTGWVQNQGILPGMVWQDRDVYLQGPIWA
KIPHTDGNFHPSPLMGGFGMKHPPPQILIKNTPVPADPPTAFNKDKLNSFITQYSTGQVSVE
IEWELQKENSKRWNPEIQYTSNYYKSNNVEFAVNTEGVYSEPRPIGTRYLTRNL
 (SEQ ID NO: 5)

In some embodiments, the peptide is not at the N- or C-terminus of the AAV capsid protein.

In some embodiments, the peptide is positioned immediately after a position corresponding to amino acid 588 of SEQ ID NO: 5.

The terms "corresponding to", "reference to" and "relative to" when used in the context of the numbering of a given amino acid or polynucleotide sequence may refer to the numbering of the residues of a specified reference sequence when the given amino acid or polynucleotide sequence is compared to the reference sequence. In other words, the residue number or residue position of a given polymer is designated with respect to the reference sequence rather than by the actual numerical position of the residue within the given amino acid or polynucleotide sequence. For example, a given amino acid sequence, such as that of an AAV capsid protein, can be aligned to a reference sequence by introducing gaps to optimise residue matches between the two sequences. In these cases, although the gaps are present, the numbering of the residue in the given amino acid or polynucleotide sequence is made with respect to the reference sequence to which it has been aligned.

Example AAV capsid sequences that comprise the peptide of the disclosure are: 
MAADGYLPDWLEDNLSEGIREWWALKPGAPQPKANQQHQDNARGLVLPGYKYLGPGNGLDKG
EPVNAADAAALEHDKAYDQQLKAGDNPYLKYNHADAEFQERLKEDTSFGGNLGRAVFQAKKR
LLEPLGLVEEAAKTAPGKKRPVEQSPQEPDSSAGIGKSGAQPAKKRLNFGQTGDTESVPDPQ
PIGEPPAAPSGVGSLTMASGGGAPVADNNEGADGVGSSSGNWHCDSQWLGDRVITTSTRTWA
LPTYNNHLYKQISNSTSGGSSNDNAYFGYSTPWGYFDFNRFHCHFSPRDWQRLINNNWGFRP
KRLNFKLFNIQVKEVTDNNGVKTIANNLTSTVQVFTDSDYQLPYVLGSAHEGCLPPFPADVF
MIPQYGYLTLNDGSQAVGRSSFYCLEYFPSQMLRTGNNFQFSYEFENVPFHSSYAHSQSLDR
LMNPLIDQYLYYLSKTINGSGQNQQTLKFSVAGPSNMAVQGRNYIPGPSYRQQRVSTTVTQN
NNSEFAWPGASSWALNGRNSLMNPGPAMASHKEGEDRFFPLSGSLIFGKQGTGRDNVDADKV
MITNEEEIKTTNPVATESYGQVATNHQSAQPGVPGRFAQAQTGWVQNQGILPGMVWQDRDVY
LQGPIWAKIPHTDGNFHPSPLMGGFGMKHPPPQILIKNTPVPADPPTAFNKDKLNSFITQYS
TGQVSVEIEWELQKENSKRWNPEIQYTSNYYKSNNVEFAVNTEGVYSEPRPIGTRYLTRNL
 (SEQ ID NO: 6) 
MAADGYLPDWLEDNLSEGIREWWALKPGAPQPKANQQHQDNARGLVLPGYKYLGPGNGLDKG
EPVNAADAAALEHDKAYDQQLKAGDNPYLKYNHADAEFQERLKEDTSFGGNLGRAVFQAKKR
LLEPLGLVEEAAKTAPGKKRPVEQSPQEPDSSAGIGKSGAQPAKKRLNFGQTGDTESVPDPQ
PIGEPPAAPSGVGSLTMASGGGAPVADNNEGADGVGSSSGNWHCDSQWLGDRVITTSTRTWA
LPTYNNHLYKQISNSTSGGSSNDNAYFGYSTPWGYFDFNRFHCHFSPRDWQRLINNNWGFRP
KRLNFKLFNIQVKEVTDNNGVKTIANNLTSTVQVFTDSDYQLPYVLGSAHEGCLPPFPADVF
MIPQYGYLTLNDGSQAVGRSSFYCLEYFPSQMLRTGNNFQFSYEFENVPFHSSYAHSQSLDR
LMNPLIDQYLYYLSKTINGSGQNQQTLKFSVAGPSNMAVQGRNYIPGPSYRQQRVSTTVTQN
NNSEFAWPGASSWALNGRNSLMNPGPAMASHKEGEDRFFPLSGSLIFGKQGTGRDNVDADKV
 
MITNEEEIKTTNPVATESYGQVATNHQSAQAGPGVPGRFAQAQTGWVQNQGILPGMVWQDRD
VYLQGPIWAKIPHTDGNFHPSPLMGGFGMKHPPPQILIKNTPVPADPPTAFNKDKLNSFITQ
YSTGQVSVEIEWELQKENSKRWNPEIQYTSNYYKSNNVEFAVNTEGVYSEPRPIGTRYLTRN
L
 (SEQ ID NO: 7) 
MAADGYLPDWLEDNLSEGIREWWALKPGAPQPKANQQHQDNARGLVLPGYKYLGPGNGLDKG
EPVNAADAAALEHDKAYDQQLKAGDNPYLKYNHADAEFQERLKEDTSFGGNLGRAVFQAKKR
LLEPLGLVEEAAKTAPGKKRPVEQSPQEPDSSAGIGKSGAQPAKKRLNFGQTGDTESVPDPQ
PIGEPPAAPSGVGSLTMASGGGAPVADNNEGADGVGSSSGNWHCDSQWLGDRVITTSTRTWA
LPTYNNHLYKQISNSTSGGSSNDNAYFGYSTPWGYFDFNRFHCHFSPRDWQRLINNNWGFRP
KRLNFKLFNIQVKEVTDNNGVKTIANNLTSTVQVFTDSDYQLPYVLGSAHEGCLPPFPADVF
MIPQYGYLTLNDGSQAVGRSSFYCLEYFPSQMLRTGNNFQFSYEFENVPFHSSYAHSQSLDR
LMNPLIDQYLYYLSKTINGSGQNQQTLKFSVAGPSNMAVQGRNYIPGPSYRQQRVSTTVTQN
NNSEFAWPGASSWALNGRNSLMNPGPAMASHKEGEDRFFPLSGSLIFGKQGTGRDNVDADKV
MITNEEEIKTTNPVATESYGQVATNHQSAQNGVRSVGAQAQTGWVQNQGILPGMVWQDRDVY
LQGPIWAKIPHTDGNFHPSPLMGGFGMKHPPPQILIKNTPVPADPPTAFNKDKLNSFITQYS
TGQVSVEIEWELQKENSKRWNPEIQYTSNYYKSNNVEFAVNTEGVYSEPRPIGTRYLTRNL
 (SEQ ID NO: 8) 
MAADGYLPDWLEDNLSEGIREWWALKPGAPQPKANQQHQDNARGLVLPGYKYLGPGNGLDKG
EPVNAADAAALEHDKAYDQQLKAGDNPYLKYNHADAEFQERLKEDTSFGGNLGRAVFQAKKR
LLEPLGLVEEAAKTAPGKKRPVEQSPQEPDSSAGIGKSGAQPAKKRLNFGQTGDTESVPDPQ
PIGEPPAAPSGVGSLTMASGGGAPVADNNEGADGVGSSSGNWHCDSQWLGDRVITTSTRTWA
LPTYNNHLYKQISNSTSGGSSNDNAYFGYSTPWGYFDFNRFHCHFSPRDWQRLINNNWGFRP
KRLNFKLFNIQVKEVTDNNGVKTIANNLTSTVQVFTDSDYQLPYVLGSAHEGCLPPFPADVF
MIPQYGYLTLNDGSQAVGRSSFYCLEYFPSQMLRTGNNFQFSYEFENVPFHSSYAHSQSLDR
LMNPLIDQYLYYLSKTINGSGQNQQTLKFSVAGPSNMAVQGRNYIPGPSYRQQRVSTTVTQN
NNSEFAWPGASSWALNGRNSLMNPGPAMASHKEGEDRFFPLSGSLIFGKQGTGRDNVDADKV
MITNEEEIKTTNPVATESYGQVATNHQSAQAGNGVRSVGAQAQTGWVQNQGILPGMVWQDRD
VYLQGPIWAKIPHTDGNFHPSPLMGGFGMKHPPPQILIKNTPVPADPPTAFNKDKLNSFITQ
YSTGQVSVEIEWELQKENSKRWNPEIQYTSNYYKSNNVEFAVNTEGVYSEPRPIGTRYLTRN
L
 (SEQ ID NO: 9)

In some embodiments, the capsid protein comprises or consists of an amino acid sequence that has at least 75%, 80%, 85%, 90%, 95%, 96%, 97%, 98% or 99% sequence identity to SEQ ID NO: 6. In some embodiments, the capsid protein comprises or consists of an amino acid sequence that has at least 75%, 80%, 85%, 90%, 95%, 96%, 97%, 98% or 99% sequence identity to SEQ ID NO: 7.

In some embodiments, the capsid protein comprises or consists of the amino acid sequence of SEQ ID NO: 6. In some embodiments, the capsid protein comprises or consists of the amino acid sequence of SEQ ID NO: 7.

In some embodiments, the capsid protein comprises or consists of an amino acid sequence that has at least 75%, 80%, 85%, 90%, 95%, 96%, 97%, 98% or 99% sequence identity to SEQ ID NO: 8. In some embodiments, the capsid protein comprises or consists of an amino acid sequence that has at least 75%, 80%, 85%, 90%, 95%, 96%, 97%, 98% or 99% sequence identity to SEQ ID NO: 9.

In some embodiments, the capsid protein comprises or consists of the amino acid sequence of SEQ ID NO: 8. In some embodiments, the capsid protein comprises or consists of the amino acid sequence of SEQ ID NO: 9.

In some embodiments, the capsid protein further comprises the mutation A587G, wherein the amino acids are numbered with reference to SEQ ID NO: 5. In some embodiments, the capsid protein further comprises the mutation Q592A, wherein the amino acids are numbered with reference to SEQ ID NO: 5. In some embodiments, the capsid protein further comprises the mutations A587G and Q592A, wherein the amino acids are numbered with reference to SEQ ID NO: 5.

In some embodiments, the capsid protein further comprises the mutation W503A, wherein the amino acids are numbered with reference to SEQ ID NO: 5.

In some embodiments, the capsid protein further comprises the mutations A587G and W503A, wherein the amino acids are numbered with reference to SEQ ID NO: 5. In some embodiments, the capsid protein further comprises the mutations Q592A and W503A, wherein the amino acids are numbered with reference to SEQ ID NO: 5. In some embodiments, the capsid protein further comprises the mutations A587G, Q592A and W503A, wherein the amino acids are numbered with reference to SEQ ID NO: 5.

In some embodiments, the capsid protein further comprises the mutation K449R, wherein the amino acids are numbered with reference to SEQ ID NO: 5.

In some embodiments, the capsid protein further comprises the mutations A587G, Q592A, W503A and K449R, wherein the amino acids are numbered with reference to SEQ ID NO: 5.

Example AAV capsid proteins of the invention are: 
MAADGYLPDWLEDNLSEGIREWWALKPGAPQPKANQQHQDNARGLVLPGYKYLGPGNGLDKG
EPVNAADAAALEHDKAYDQQLKAGDNPYLKYNHADAEFQERLKEDTSFGGNLGRAVFQAKKR
LLEPLGLVEEAAKTAPGKKRPVEQSPQEPDSSAGIGKSGAQPAKKRLNFGQTGDTESVPDPQ
PIGEPPAAPSGVGSLTMASGGGAPVADNNEGADGVGSSSGNWHCDSQWLGDRVITTSTRTWA
LPTYNNHLYKQISNSTSGGSSNDNAYFGYSTPWGYFDFNRFHCHFSPRDWQRLINNNWGFRP
KRLNFKLFNIQVKEVTDNNGVKTIANNLTSTVQVFTDSDYQLPYVLGSAHEGCLPPFPADVF
MIPQYGYLTLNDGSQAVGRSSFYCLEYFPSQMLRTGNNFQFSYEFENVPFHSSYAHSQSLDR
LMNPLIDQYLYYLSRTINGSGQNQQTLKFSVAGPSNMAVQGRNYIPGPSYRQQRVSTTVTQN
NNSEFAWPGASSWALNGRNSLMNPGPAMASHKEGEDRFFPLSGSLIFGKQGTGRDNVDADKV
MITNEEEIKTTNPVATESYGQVATNHQSGQAGPGVPGRFAQAATGWVQNQGILPGMVWQDRD
VYLQGPIWAKIPHTDGNFHPSPLMGGFGMKHPPPQILIKNTPVPADPPTAFNKDKLNSFITQ
 
YSTGQVSVEIEWELQKENSKRWNPEIQYTSNYYKSNNVEFAVNTEGVYSEPRPIGTRYLTRN
L
 (SEQ ID NO: 10 - Se2) 
MAADGYLPDWLEDNLSEGIREWWALKPGAPQPKANQQHQDNARGLVLPGYKYLGPGNGLDKG
EPVNAADAAALEHDKAYDQQLKAGDNPYLKYNHADAEFQERLKEDTSFGGNLGRAVFQAKKR
LLEPLGLVEEAAKTAPGKKRPVEQSPQEPDSSAGIGKSGAQPAKKRLNFGQTGDTESVPDPQ
PIGEPPAAPSGVGSLTMASGGGAPVADNNEGADGVGSSSGNWHCDSQWLGDRVITTSTRTWA
LPTYNNHLYKQISNSTSGGSSNDNAYFGYSTPWGYFDFNRFHCHFSPRDWQRLINNNWGFRP
KRLNFKLFNIQVKEVTDNNGVKTIANNLTSTVQVFTDSDYQLPYVLGSAHEGCLPPFPADVF
MIPQYGYLTLNDGSQAVGRSSFYCLEYFPSQMLRTGNNFQFSYEFENVPFHSSYAHSQSLDR
LMNPLIDQYLYYLSKTINGSGQNQQTLKFSVAGPSNMAVQGRNYIPGPSYRQQRVSTTVTQN
NNSEFAAPGASSWALNGRNSLMNPGPAMASHKEGEDRFFPLSGSLIFGKQGTGRDNVDADKV
MITNEEEIKTTNPVATESYGQVATNHQSGQAGNGVRSVGAQAATGWVQNQGILPGMVWQDRD
VYLQGPIWAKIPHTDGNFHPSPLMGGFGMKHPPPQILIKNTPVPADPPTAFNKDKLNSFITQ
YSTGQVSVEIEWELQKENSKRWNPEIQYTSNYYKSNNVEFAVNTEGVYSEPRPIGTRYLTRN
L
 (SEQ ID NO: 11 - Se1)

In some embodiments, the capsid protein comprises or consists of an amino acid sequence that has at least 75%, 80%, 85%, 90%, 95%, 96%, 97%, 98% or 99% sequence identity to SEQ ID NO: 10.

In some embodiments, the capsid protein comprises or consists of the amino acid sequence of SEQ ID NO: 10.

In some embodiments, the capsid protein comprises or consists of an amino acid sequence that has at least 75%, 80%, 85%, 90%, 95%, 96%, 97%, 98% or 99% sequence identity to SEQ ID NO: 11.

In some embodiments, the capsid protein comprises or consists of the amino acid sequence of SEQ ID NO: 11. 
ATGGCTGCCGATGGTTATCTTCCAGATTGGCTCGAGGACAACCTTAGTGAAGGAATTCGCGA
GTGGTGGGCTTTGAAACCTGGAGCCCCTCAACCCAAGGCAAATCAACAACATCAAGACAACG
CTCGAGGTCTTGTGCTTCCGGGTTACAAATACCTTGGACCCGGCAACGGACTCGACAAGGGG
GAGCCGGTCAACGCAGCAGACGCGGCGGCCCTCGAGCACGACAAGGCCTACGACCAGCAGCT
CAAGGCCGGAGACAACCCGTACCTCAAGTACAACCACGCCGACGCCGAGTTCCAGGAGCGGC
TCAAAGAAGATACGTCTTTTGGGGGCAACCTCGGGCGAGCAGTCTTCCAGGCCAAAAAGAGG
CTTCTTGAACCTCTTGGTCTGGTTGAGGAAGCGGCTAAGACGGCTCCTGGAAAGAAGAGGCC
TGTAGAGCAGTCTCCTCAGGAACCGGACTCCTCCGCGGGTATTGGCAAATCGGGTGCACAGC
CCGCTAAAAAGAGACTCAATTTCGGTCAGACTGGCGACACAGAGTCAGTCCCAGACCCTCAA
CCAATCGGAGAACCTCCCGCAGCCCCCTCAGGTGTGGGATCTCTTACAATGGCTTCAGGTGG
TGGCGCACCAGTGGCAGACAATAACGAAGGTGCCGATGGAGTGGGTAGTTCCTCGGGAAATT
GGCATTGCGATTCCCAATGGCTGGGGGACAGAGTCATCACCACCAGCACCCGAACCTGGGCC
CTGCCCACCTACAACAATCACCTCTACAAGCAAATCTCCAACAGCACATCTGGAGGATCTTC
AAATGACAACGCCTACTTCGGCTACAGCACCCCCTGGGGGTATTTTGACTTCAACAGATTCC
ACTGCCACTTCTCACCACGTGACTGGCAGCGACTCATCAACAACAACTGGGGATTCCGGCCT
AAGCGACTCAACTTCAAGCTCTTCAACATTCAGGTCAAAGAGGTTACGGACAACAATGGAGT
 
CAAGACCATCGCCAATAACCTTACCAGCACGGTCCAGGTCTTCACGGACTCAGACTATCAGC
TCCCGTACGTGCTCGGGTCGGCTCACGAGGGCTGCCTCCCGCCGTTCCCAGCGGACGTTTTC
ATGATTCCTCAGTACGGGTATCTGACGCTTAATGATGGAAGCCAGGCCGTGGGTCGTTCGTC
CTTTTACTGCCTGGAATATTTCCCGTCGCAAATGCTAAGAACGGGTAACAACTTCCAGTTCA
GCTACGAGTTTGAGAACGTACCTTTCCATAGCAGCTACGCTCACAGCCAAAGCCTGGACCGA
CTAATGAATCCACTCATCGACCAATACTTGTACTATCTCTCTAGAACTATTAACGGTTCTGG
ACAGAATCAACAAACGCTAAAATTCAGTGTGGCCGGACCCAGCAACATGGCTGTCCAGGGAA
GAAACTACATACCTGGACCCAGCTACCGACAACAACGTGTCTCAACCACTGTGACTCAAAAC
AACAACAGCGAATTTGCTTGGCCTGGAGCTTCTTCTTGGGCTCTCAATGGACGTAATAGCTT
GATGAATCCTGGACCTGCTATGGCCAGCCACAAAGAAGGAGAGGACCGTTTCTTTCCTTTGT
CTGGATCTTTAATTTTTGGCAAACAAGGTACCGGCAGAGACAACGTGGATGCGGACAAAGTC
ATGATAACCAACGAAGAAGAAATTAAAACTACTAACCCGGTAGCAACGGAGTCCTATGGACA
AGTGGCCACAAACCACCAGAGTGGCCAAGCAGGCCCTGGTGTTCCGGGGCGGTTTGCCCAGG
CGGCCACCGGTTGGGTTCAAAACCAAGGAATACTTCCGGGTATGGTTTGGCAGGACAGAGAT
GTGTACCTGCAAGGACCCATTTGGGCCAAAATTCCTCACACGGACGGCAACTTTCACCCTTC
TCCGCTGATGGGAGGGTTTGGAATGAAGCACCCGCCTCCTCAGATCCTCATCAAAAACACAC
CTGTACCTGCGGATCCTCCAACGGCCTTCAACAAGGACAAGCTGAACTCTTTCATCACCCAG
TATTCTACTGGCCAAGTCAGCGTGGAGATCGAGTGGGAGCTGCAGAAGGAAAACAGCAAGCG
CTGGAACCCGGAGATCCAGTACACTTCCAACTATTACAAGTCTAATAATGTTGAATTTGCTG
TTAATACTGAAGGTGTATATAGTGAACCCCGCCCCATTGGCACCAGATACCTGACTCGTAAT
CTGTAA
 (SEQ ID NO: 12 - Se2) 
ATGGCTGCCGATGGTTATCTTCCAGATTGGCTCGAGGACAACCTTAGTGAAGGAATTCGCGA
GTGGTGGGCTTTGAAACCTGGAGCCCCTCAACCCAAGGCAAATCAACAACATCAAGACAACG
CTCGAGGTCTTGTGCTTCCGGGTTACAAATACCTTGGACCCGGCAACGGACTCGACAAGGGG
GAGCCGGTCAACGCAGCAGACGCGGCGGCCCTCGAGCACGACAAGGCCTACGACCAGCAGCT
CAAGGCCGGAGACAACCCGTACCTCAAGTACAACCACGCCGACGCCGAGTTCCAGGAGCGGC
TCAAAGAAGATACGTCTTTTGGGGGCAACCTCGGGCGAGCAGTCTTCCAGGCCAAAAAGAGG
CTTCTTGAACCTCTTGGTCTGGTTGAGGAAGCGGCTAAGACGGCTCCTGGAAAGAAGAGGCC
TGTAGAGCAGTCTCCTCAGGAACCGGACTCCTCCGCGGGTATTGGCAAATCGGGTGCACAGC
CCGCTAAAAAGAGACTCAATTTCGGTCAGACTGGCGACACAGAGTCAGTCCCAGACCCTCAA
CCAATCGGAGAACCTCCCGCAGCCCCCTCAGGTGTGGGATCTCTTACAATGGCTTCAGGTGG
TGGCGCACCAGTGGCAGACAATAACGAAGGTGCCGATGGAGTGGGTAGTTCCTCGGGAAATT
GGCATTGCGATTCCCAATGGCTGGGGGACAGAGTCATCACCACCAGCACCCGAACCTGGGCC
CTGCCCACCTACAACAATCACCTCTACAAGCAAATCTCCAACAGCACATCTGGAGGATCTTC
AAATGACAACGCCTACTTCGGCTACAGCACCCCCTGGGGGTATTTTGACTTCAACAGATTCC
ACTGCCACTTCTCACCACGTGACTGGCAGCGACTCATCAACAACAACTGGGGATTCCGGCCT
AAGCGACTCAACTTCAAGCTCTTCAACATTCAGGTCAAAGAGGTTACGGACAACAATGGAGT
CAAGACCATCGCCAATAACCTTACCAGCACGGTCCAGGTCTTCACGGACTCAGACTATCAGC
TCCCGTACGTGCTCGGGTCGGCTCACGAGGGCTGCCTCCCGCCGTTCCCAGCGGACGTTTTC
ATGATTCCTCAGTACGGGTATCTGACGCTTAATGATGGAAGCCAGGCCGTGGGTCGTTCGTC
CTTTTACTGCCTGGAATATTTCCCGTCGCAAATGCTAAGAACGGGTAACAACTTCCAGTTCA
GCTACGAGTTTGAGAACGTACCTTTCCATAGCAGCTACGCTCACAGCCAAAGCCTGGACCGA
CTAATGAATCCACTCATCGACCAATACTTGTACTATCTCTCAAAGACTATTAACGGTTCTGG
ACAGAATCAACAAACGCTAAAATTCAGTGTGGCCGGACCCAGCAACATGGCTGTCCAGGGAA
GAAACTACATACCTGGACCCAGCTACCGACAACAACGTGTCTCAACCACTGTGACTCAAAAC
AACAACAGCGAATTTGCTGCGCCTGGAGCTTCTTCTTGGGCTCTCAATGGACGTAATAGCTT
GATGAATCCTGGACCTGCTATGGCCAGCCACAAAGAAGGAGAGGACCGTTTCTTTCCTTTGT
CTGGATCTTTAATTTTTGGCAAACAAGGAACTGGAAGAGACAACGTGGATGCGGACAAAGTC
ATGATAACCAACGAAGAAGAAATTAAAACTACTAACCCGGTAGCAACGGAGTCCTATGGACA
AGTGGCCACAAACCACCAGAGTGGCCAAGCAGGCAATGGTGTTCGTTCTGTGGGTGCCCAGG
CGGCCACCGGCTGGGTTCAAAACCAAGGAATACTTCCGGGTATGGTTTGGCAGGACAGAGAT
GTGTACCTGCAAGGACCCATTTGGGCCAAAATTCCTCACACGGACGGCAACTTTCACCCTTC
TCCGCTGATGGGAGGGTTTGGAATGAAGCACCCGCCTCCTCAGATCCTCATCAAAAACACAC
 
CTGTACCTGCGGATCCTCCAACGGCCTTCAACAAGGACAAGCTGAACTCTTTCATCACCCAG
TATTCTACTGGCCAAGTCAGCGTGGAGATCGAGTGGGAGCTGCAGAAGGAAAACAGCAAGCG
CTGGAACCCGGAGATCCAGTACACTTCCAACTATTACAAGTCTAATAATGTTGAATTTGCTG
TTAATACTGAAGGTGTATATAGTGAACCCCGCCCCATTGGCACCAGATACCTGACTCGTAAT
CTGTAA
 (SEQ ID NO: 13 - Se1)

In some embodiments, the capsid protein is encoded by a nucleotide sequence that has at least 75%, 80%, 85%, 90%, 95%, 96%, 97%, 98% or 99% sequence identity to SEQ ID NO: 12.

In some embodiments, the capsid protein is encoded by the nucleotide sequence of SEQ ID NO: 12.

In some embodiments, the capsid protein is encoded by a nucleotide sequence that has at least 75%, 80%, 85%, 90%, 95%, 96%, 97%, 98% or 99% sequence identity to SEQ ID NO: 13.

In some embodiments, the capsid protein is encoded by the nucleotide sequence of SEQ ID NO: 13.

In one aspect, the invention provides a polynucleotide comprising or consisting of a nucleotide sequence that has at least 75%, 80%, 85%, 90%, 95%, 96%, 97%, 98% or 99% sequence identity to SEQ ID NO: 12.

In one aspect, the invention provides a polynucleotide comprising or consisting of the nucleotide sequence of SEQ ID NO: 12.

In one aspect, the invention provides a polynucleotide comprising or consisting of a nucleotide sequence that has at least 75%, 80%, 85%, 90%, 95%, 96%, 97%, 98% or 99% sequence identity to SEQ ID NO: 13.

In one aspect, the invention provides a polynucleotide comprising or consisting of the nucleotide sequence of SEQ ID NO: 13.

### AAV genomes

Typically, the AAV genome of a naturally derived serotype, isolate or clade of AAV comprises at least one inverted terminal repeat sequence (ITR). An ITR sequence acts in cis to provide a functional origin of replication and allows for integration and excision of the vector from the genome of a cell. The AAV genome may also comprise packaging genes, such as rep and/or cap genes which encode packaging functions for an AAV particle. The rep gene encodes one or more of the proteins Rep78, Rep68, Rep52 and Rep40 or variants thereof. The cap gene encodes one or more capsid proteins such as VP1, VP2 and VP3 or variants thereof. These proteins make up the capsid of an AAV particle.

A promoter may be operably linked to each of the packaging genes. Specific examples of such promoters include the p5, p19 and p40 promoters (Laughlin et al. (1979) Proc. Natl. Acad. Sci. USA 76: 5567-5571). For example, the p5 and p19 promoters are generally used to express the rep gene, while the p40 promoter is generally used to express the cap gene.

The AAV genome used in the AAV vector of the invention may therefore be the full genome of a naturally occurring AAV. For example, a vector comprising a full AAV genome may be used to prepare an AAV vector or vector particle in vitro. However, while such a vector may in principle be administered to patients, this will rarely be done in practice. Preferably, the AAV genome will be derivatised for the purpose of administration to patients. Such derivatisation is standard in the art and the invention encompasses the use of any known derivative of an AAV genome, and derivatives which could be generated by applying techniques known in the art. Derivatisation of the AAV genome and of the AAV capsid are reviewed in Coura and Nardi (2007) Virology Journal 4: 99, and in Choi et al. and Wu et al., referenced above.

Derivatives of an AAV genome include any truncated or modified forms of an AAV genome which allow for expression of a transgene from an AAV in vivo. Typically, it is possible to truncate the AAV genome significantly to include minimal viral sequence yet retain the above function. This is preferred for safety reasons to reduce the risk of recombination of the vector with wild-type virus, and also to avoid triggering a cellular immune response by the presence of viral gene proteins in the target cell.

Typically, a derivative will include at least one inverted terminal repeat sequence (ITR), preferably more than one ITR, such as two ITRs or more. One or more of the ITRs may be derived from AAV genomes having different serotypes, or may be a chimeric or mutant ITR. A preferred mutant ITR is one having a deletion of a trs (terminal resolution site). This deletion allows for continued replication of the genome to generate a single-stranded genome which contains both coding and complementary sequences, i.e. a self-complementary AAV genome. This allows for bypass of DNA replication in the target cell, and so enables accelerated transgene expression.

In some embodiments, the AAV comprises at least one, such as two, AAV serotype 1, 2, 3, 4, 5, 6, 7, 8, 9, 10 or 11 ITRs. Preferably, the AAV comprises at least one, such as two, AAV serotype 2 ITRs.

The one or more ITRs will preferably flank the nucleotide sequence of interest (which may also be referred to as a transgene) at either end. The inclusion of one or more ITRs is preferred to aid concatamer formation in the nucleus of a host cell, for example following the conversion of single-stranded vector DNA into double-stranded DNA by the action of host cell DNA polymerases. The formation of such episomal concatamers protects the vector construct during the life of the host cell, thereby allowing for prolonged expression of the transgene in vivo.

In preferred embodiments, ITR elements will be the only sequences retained from the native AAV genome in the derivative. Thus, a derivative will preferably not include the rep and/or cap genes of the native genome and any other sequences of the native genome. This is preferred for the reasons described above, and also to reduce the possibility of integration of the vector into the host cell genome. Additionally, reducing the size of the AAV genome allows for increased flexibility in incorporating other sequence elements (such as regulatory elements) within the vector in addition to the transgene.

The following portions could therefore be removed in a derivative of the invention: one inverted terminal repeat (ITR) sequence, the replication (rep) and capsid (cap) genes. However, in some embodiments, derivatives may additionally include one or more rep and/or cap genes or other viral sequences of an AAV genome. Naturally occurring AAV integrates with a high frequency at a specific site on human chromosome 19, and shows a negligible frequency of random integration, such that retention of an integrative capacity in the vector may be tolerated in a therapeutic setting.

The invention additionally encompasses the provision of sequences of an AAV genome in a different order and configuration to that of a native AAV genome. The invention also encompasses the replacement of one or more AAV sequences or genes with sequences from another virus or with chimeric genes composed of sequences from more than one virus. Such chimeric genes may be composed of sequences from two or more related viral proteins of different viral species.

The AAV particles of the invention include transcapsidated forms wherein an AAV genome or derivative having an ITR of one serotype is packaged in the capsid of a different serotype. The AAV particles of the invention also include mosaic forms wherein a mixture of capsid proteins from two or more different serotypes makes up the viral capsid. The AAV particle also includes chemically modified forms bearing ligands adsorbed to the capsid surface. For example, such ligands may include antibodies for targeting a particular cell surface receptor.

The AAV may comprise multiple copies (e.g., 2, 3 etc.) of the polynucleotide referred to herein.

### PROMOTERS AND REGULATORY SEQUENCES

The AAV particle of the invention may also include elements allowing for the expression of the nucleotide sequence of interest in vitro or in vivo. These may be referred to as expression control sequences. Thus, the AAV typically comprises expression control sequences (e.g. comprising a promoter sequence) operably linked to the nucleotide sequence of interest.

Any suitable promoter may be used, the selection of which may be readily made by the skilled person. The promoter sequence may be constitutively active (i.e. operational in any host cell background), or alternatively may be active only in a specific host cell environment, thus allowing for targeted expression of the nucleotide sequence of interest in a particular cell type (e.g. a tissue-specific promoter such as an endothelial specific promoter). The promoter may show inducible expression in response to presence of another factor, for example a factor present in a host cell. In any event, where the vector is administered for therapy, it is preferred that the promoter should be functional in the target cell background.

In some embodiments, it is preferred that the promoter shows neural cell expression in order to allow for the nucleotide sequence of interest to only be expressed in neural cell populations. Thus, expression from the promoter may be neural-cell specific.

In other embodiments, it is preferred that the promoter shows endothelial cell expression in order to allow for the nucleotide sequence of interest to only be expressed in endothelial cell populations. Thus, expression from the promoter may be endothelial-cell specific.

Example promoters, which are not cell specific, include the chicken beta-actin (CBA) promoter, optionally in combination with a cytomegalovirus (CMV) enhancer element. An example promoter for use in the invention is a CAG promoter.

The AAV vector of the invention may also comprise one or more additional regulatory sequences which may act pre- or post-transcriptionally. The regulatory sequence may be part of the native transgene locus or may be a heterologous regulatory sequence. The AAV vector of the invention may comprise portions of the 5'-UTR or 3'-UTR from the native transgene transcript.

Regulatory sequences are any sequences which facilitate expression of the transgene, i.e. act to increase expression of a transcript, improve nuclear export of mRNA or enhance its stability. Such regulatory sequences include for example enhancer elements, post-transcriptional regulatory elements and polyadenylation sites. An example of a polyadenylation site is the Bovine Growth Hormone poly-A signal.

In the context of the AAV of the invention, such regulatory sequences may be cis-acting.

An example of a post-transcriptional regulatory element for use in a AAV of the invention is the woodchuck hepatitis post-transcriptional regulatory element (WPRE) or a variant thereof.

Another regulatory sequence which may be used in a AAV vector of the invention is a scaffold-attachment region (SAR). Additional regulatory sequences may be readily selected by the skilled person.

### NON-VIRAL PARTICLE

In one aspect, the invention provides use of a peptide for targeting a non-viral particle to cells of the brain or central nervous system, wherein the peptide:
(a) comprises or consists of the amino acid sequence PGVPGRF (SEQ ID NO: 1), or a variant thereof having up to two amino acid substitutions, additions or deletions; or
(b) comprises or consists of the amino acid sequence NGVRSVG (SEQ ID NO: 3), or a variant thereof having up to two amino acid substitutions, additions or deletions.

In one aspect, the invention provides use of a peptide for targeting a non-viral particle to cells of the brain or central nervous system, wherein the peptide:
(a) comprises or consists of the amino acid sequence AGPGVPGRF (SEQ ID NO: 2), or a variant thereof having up to two amino acid substitutions, additions or deletions; or
(b) comprises or consists of the amino acid sequence AGNGVRSVG (SEQ ID NO: 4), or a variant thereof having up to two amino acid substitutions, additions or deletions.

In one aspect, the invention provides use of a peptide for targeting a non-viral particle to cells of the peripheral nervous system, wherein the peptide:
(a) comprises or consists of the amino acid sequence PGVPGRF (SEQ ID NO: 1), or a variant thereof having up to two amino acid substitutions, additions or deletions; or
(b) comprises or consists of the amino acid sequence NGVRSVG (SEQ ID NO: 3), or a variant thereof having up to two amino acid substitutions, additions or deletions.

In one aspect, the invention provides use of a peptide for targeting a non-viral particle to cells of the peripheral nervous system, wherein the peptide:
(a) comprises or consists of the amino acid sequence AGPGVPGRF (SEQ ID NO: 2), or a variant thereof having up to two amino acid substitutions, additions or deletions; or
(b) comprises or consists of the amino acid sequence AGNGVRSVG (SEQ ID NO: 4), or a variant thereof having up to two amino acid substitutions, additions or deletions.

In one aspect, the invention provides use of a peptide for increasing the efficiency of transfection of cells of the brain by a non-viral particle, wherein the peptide:
(a) comprises or consists of the amino acid sequence PGVPGRF (SEQ ID NO: 1), or a variant thereof having up to two amino acid substitutions, additions or deletions; or
(b) comprises or consists of the amino acid sequence NGVRSVG (SEQ ID NO: 3), or a variant thereof having up to two amino acid substitutions, additions or deletions.

In one aspect, the invention provides use of a peptide for increasing the efficiency of transfection of cells of the brain by a non-viral particle, wherein the peptide:
(a) comprises or consists of the amino acid sequence AGPGVPGRF (SEQ ID NO: 2), or a variant thereof having up to two amino acid substitutions, additions or deletions; or
(b) comprises or consists of the amino acid sequence AGNGVRSVG (SEQ ID NO: 4), or a variant thereof having up to two amino acid substitutions, additions or deletions.

In one aspect, the invention provides use of a peptide for decreasing tropism of a non-viral particle for non-brain tissue, wherein the peptide:
(a) comprises or consists of the amino acid sequence PGVPGRF (SEQ ID NO: 1), or a variant thereof having up to two amino acid substitutions, additions or deletions; or
(b) comprises or consists of the amino acid sequence NGVRSVG (SEQ ID NO: 3), or a variant thereof having up to two amino acid substitutions, additions or deletions.

In one aspect, the invention provides use of a peptide for decreasing tropism of a non-viral particle for non-brain tissue, wherein the peptide:
(a) comprises or consists of the amino acid sequence AGPGVPGRF (SEQ ID NO: 2), or a variant thereof having up to two amino acid substitutions, additions or deletions; or
(b) comprises or consists of the amino acid sequence AGNGVRSVG (SEQ ID NO: 4), or a variant thereof having up to two amino acid substitutions, additions or deletions.

In some embodiments, the non-viral particle is a nanoparticle.

In some embodiments, the non-viral particle is a lipid nanoparticle.

In some embodiments, the non-viral particle is a liposome.

### VARIANTS, DERIVATIVES, ANALOGUES, HOMOLOGUES AND FRAGMENTS

In addition to the specific proteins and nucleotides mentioned herein, the invention also encompasses variants, derivatives, analogues, homologues and fragments thereof.

In the context of the invention, a "variant" of any given sequence is a sequence in which the specific sequence of residues (whether amino acid or nucleic acid residues) has been modified in such a manner that the polypeptide or polynucleotide in question retains at least one of its endogenous functions. A variant sequence can be obtained by addition, deletion, substitution, modification, replacement and/or variation of at least one residue present in the naturally occurring polypeptide or polynucleotide.

The term "derivative" as used herein in relation to proteins or polypeptides of the invention includes any substitution of, variation of, modification of, replacement of, deletion of and/or addition of one (or more) amino acid residues from or to the sequence, providing that the resultant protein or polypeptide retains at least one of its endogenous functions.

The term "analogue" as used herein in relation to polypeptides or polynucleotides includes any mimetic, that is, a chemical compound that possesses at least one of the endogenous functions of the polypeptides or polynucleotides which it mimics.

Typically, amino acid substitutions may be made, for example from 1, 2 or 3, to 10 or 20 substitutions, provided that the modified sequence retains the required activity or ability. Amino acid substitutions may include the use of non-naturally occurring analogues.

Proteins used in the invention may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the endogenous function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

Conservative substitutions may be made, for example according to the table below. Amino acids in the same block in the second column and preferably in the same line in the third column may be substituted for each other:

| | | |
|---|---|---|
| ALIPHATIC | Non-polar | GAP |
| | | ILV |
| | Polar - uncharged | C S T M |
| | | NQ |
| | Polar - charged | D E |
| | | KRH |
| AROMA TIC | | F W Y |

The term "homologue" as used herein means an entity having a certain homology with the wild type amino acid sequence or the wild type nucleotide sequence. The term "homology" can be equated with "identity".

In the present context, a homologous sequence is taken to include an amino acid sequence which may be at least 50%, 55%, 65%, 75%, 85% or 90% identical, preferably at least 95%, 96% or 97% or 98% or 99% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 50%, 55%, 65%, 75%, 85% or 90% identical, preferably at least 95%, 96% or 97% or 98% or 99% identical to the subject sequence. Although homology can also be considered in terms of similarity, in the context of the present invention it is preferred to express homology in terms of sequence identity.

Preferably, reference to a sequence which has a percent identity to any one of the SEQ ID NOs detailed herein refers to a sequence which has the stated percent identity over the entire length of the SEQ ID NO referred to.

Homology comparisons can be conducted by eye, or more usually, with the aid of readily available sequence comparison programs. These commercially available computer programs can calculate percent homology or identity between two or more sequences.

Percent homology may be calculated over contiguous sequences, i.e. one sequence is aligned with the other sequence and each amino acid or nucleotide in one sequence is directly compared with the corresponding amino acid or nucleotide in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the amino acid or nucleotide sequence may cause the following residues or codons to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids or nucleotides, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum percent homology therefore firstly requires the production of an optimal alignment, taking into consideration gap penalties. A suitable computer program for carrying out such an alignment is the GCG Wisconsin Bestfit package (University of Wisconsin, USA; Devereux et al. (1984) Nucleic Acids Research 12: 387). Examples of other software that can perform sequence comparisons include, but are not limited to, the BLAST package, FASTA (Atschul et al. (1990) J. Mol. Biol. 403-410) and the GENEWORKS suite of comparison tools. Both BLAST and FASTA are available for offline and online searching. However, for some applications, it is preferred to use the GCG Bestfit program. Another tool, BLAST 2 Sequences, is also available for comparing protein and nucleotide sequences (FEMS Microbiol. Lett. (1999) 174(2):247-50; FEMS Microbiol. Lett. (1999) 177(1):187-8).

Although the final percent homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix (the default matrix for the BLAST suite of programs). GCG Wisconsin programs generally use either the public default values or a custom symbol comparison table if supplied (see the user manual for further details). For some applications, it is preferred to use the public default values for the GCG package, or in the case of other software, the default matrix, such as BLOSUM62.

Once the software has produced an optimal alignment, it is possible to calculate percent homology, preferably percent sequence identity. The software typically does this as part of the sequence comparison and generates a numerical result.

"Fragments" are also variants and the term typically refers to a selected region of the polypeptide or polynucleotide that is of interest either functionally or, for example, in an assay. "Fragment" thus refers to an amino acid or nucleic acid sequence that is a portion of a full-length polypeptide or polynucleotide.

Such variants may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

### Codon optimisation

The polynucleotides used in the invention may be codon-optimised. Codon optimisation has previously been described in WO 1999/41397 and WO 2001/79518. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available. Codon usage tables are known in the art for mammalian cells, as well as for a variety of other organisms.

### METHOD OF TREATMENT

All references herein to treatment include curative, palliative and prophylactic treatment. The treatment of mammals, particularly humans, is preferred. Both human and veterinary treatments are within the scope of the invention.

In some embodiments, the method of treatment provides the nucleotide of interest to the central nervous system and/or the peripheral nervous system of the subject.

In some embodiments, the method of treatment provides the nucleotide of interest to the cerebral cortex, caudate nucleus, thalamus and/or cerebellum of the subject.

In some embodiments, the method of treatment provides the nucleotide of interest to the forebrain, midbrain, cortex, hippocampus and/or cerebellum of the subject.

In some embodiments, the method of treatment provides the nucleotide of interest to the dorsal root ganglia and/or sympathetic ganglia.

In some embodiments, the method of treatment provides the nucleotide of interest to glial and/or neuronal cells, preferably dopaminergic neurons.

In some embodiments, the method of treatment provides an improvement in learning and/or cognitive function in the subject. Methods for measuring learning and/or cognitive function are known to the skilled person. For example, in humans the General Practitioner Assessment of Cognition (GPCOG) test may be used. Alternative cognitive tests include but are not limited to the Mini Mental State Examination (MMSE), The Six-item Cognitive Impairment Test (6CIT), Abbreviated Mental Test (AMT) and Informant Questionnaire on Cognitive Decline in the Elderly (IQCODE).

Advantageously, the present invention provides a method for treating dopaminergic neurons by systemically administering the vector particle of the invention. These neurons are vital to diverse brain functions, including: voluntary movement, reward, addiction and stress. Progressive loss of dopaminergic neurons is responsible for neurodegenerative diseases such as Parkinson's Disease.

### PHARMACEUTICAL COMPOSITIONS AND INJECTED SOLUTIONS

Although the agents for use in the invention can be administered alone, they will generally be administered in admixture with a pharmaceutical carrier, excipient or diluent, particularly for human therapy.

A "pharmaceutical composition" may refer to a preparation which is stable and in a form which is acceptable to the patient.

The medicaments, for example AAV particles, of the invention may be formulated into pharmaceutical compositions. These compositions may comprise, in addition to the medicament, a pharmaceutically acceptable carrier, diluent, excipient, buffer, stabiliser or other materials well known in the art. Such materials should be non-toxic and should not interfere with the efficacy of the active ingredient. The precise nature of the carrier or other material may be determined by the skilled person according to the route of administration, e.g. intravenous or intra-arterial.

The pharmaceutical composition is typically in liquid form. Liquid pharmaceutical compositions generally include a liquid carrier such as water, petroleum, animal or vegetable oils, mineral oil or synthetic oil. Physiological saline solution, magnesium chloride, dextrose or other saccharide solution, or glycols such as ethylene glycol, propylene glycol or polyethylene glycol may be included. In some cases, a surfactant, such as pluronic acid (PF68) 0.001% may be used. In some cases, serum albumin may be used in the composition.

For injection, the active ingredient may be in the form of an aqueous solution which is pyrogen-free, and has suitable pH, isotonicity and stability. The skilled person is well able to prepare suitable solutions using, for example, isotonic vehicles such as Sodium Chloride Injection, Ringer's Injection or Lactated Ringer's Injection. Preservatives, stabilisers, buffers, antioxidants and/or other additives may be included as required.

For delayed release, the medicament may be included in a pharmaceutical composition which is formulated for slow release, such as in microcapsules formed from biocompatible polymers or in liposomal carrier systems according to methods known in the art.

Handling of the cell therapy products is preferably performed in compliance with FACT-JACIE International Standards for cellular therapy.

### ADMINISTRATION

In some embodiments, the agent of the invention is systemically administered to the subject.

The term "systemic delivery" or "systemic administration" as used herein, means that the agent of the invention is administered into the circulatory system, e.g. to achieve broad distribution of the agent. In contrast, topical or local administration restricts the delivery of the agent to a localised area, e.g. intracerebral administration entails direct injection into the brain.

In some embodiments, the agent of the invention is administered intravenously.

In some embodiments, the agent of the invention is administered intra-arterially.

As used herein, the term "agent" may refer to the vector particle or pharmaceutical composition of the invention.

### DOSAGE

The skilled person can readily determine an appropriate dose of an agent of the invention to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. There can of course be individual instances where higher or lower dosage ranges are merited, and such are within the scope of the invention.

### SUBJECT

The term "subject" as used herein refers to either a human or non-human animal.

Examples of non-human animals include vertebrates, for example mammals, such as non-human primates (particularly higher primates), dogs, rodents (e.g. mice, rats or guinea pigs), pigs and cats. The non-human animal may be a companion animal.

Preferably, the subject is a human.

### DISEASES

Neurodegenerative diseases are diseases which primarily affect neurons. These diseases result in the progressive loss of structure or function of nerve cells (neurons) including neuronal cell death. This causes problems with movement (called ataxias), or mental functioning (called dementias).

The invention encompasses agents, compositions and methods for treating and/or delaying progression of a neurodegenerative disease in a subject. The subject may have a neurodegenerative disease, i.e. has been diagnosed as having the neurodegenerative disease or is suspected as having the neurodegenerative disease, or may be asymptomatic. The subject can have a genetic predisposition to the neurodegenerative disease. The subject may have one or more family members with a neurodegenerative disease.

In some embodiments, the neurodegenerative disease is Parkinson's disease. In some embodiments, the neurodegenerative disease is a Parkinson's disease-related disorder. Preferably, the Parkinson's disease-related disorder is Parkinson's dementia. Preferably, the Parkinson's disease-related disorder is dementia with Lewy bodies. In some embodiments, the neurodegenerative disease is lower body Parkinson's syndrome.

In some embodiments, the neurodegenerative disorder is associated with Lewy bodies.

Lewy bodies are abnormal aggregates of protein that develop inside nerve cells and may be found within the substantia nigra or within the cortex. Lewy bodies comprise alpha-synuclein associated with other proteins which may include ubiquitin, neurofilament protein and alpha B crystallin. Lewy neurites may be associated with Lewy bodies.

In some embodiments, the neurodegenerative disease is dementia with Lewy bodies.

In some embodiments, the neurodegenerative disease is multiple system atrophy. In some embodiments, the neurodegenerative disease is multisystemic atrophy cerebellar type (MSA-C).

In some embodiments, the neurodegenerative disease is fronto-temporal dementia. In some embodiments, the neurodegenerative disease is fronto-temporal dementia with Parkinson's disease.

In some embodiments, the neurodegenerative disease is Gaucher disease.

The neurodegenerative disease may be associated with defects in lysosomal storage. The invention encompasses agents, compositions and methods for treating or delaying progression of a lysosomal storage disease in a subject. The subject may have a lysosomal storage disease, i.e. has been diagnosed as having the lysosomal storage disease or is suspected as having the lysosomal storage disease, or may be asymptomatic. The subject can have a genetic predisposition to the lysosomal storage disease. The subject may have one or more family members with a lysosomal storage disease.

"Lysosome storage disease", as used herein, refers to any condition that involves defects in or disruption of lysosomal function. These defects are usually a consequence of a deficiency of an enzyme required for the metabolism of lipids, glycoproteins or mucopolysaccharides.

Gaucher disease is one of the most common lysosomal storage diseases. It is a genetic disorder in which glucocerebroside accumulates in cells and certain organs. The disease is caused by a hereditary deficiency in the enzyme glucocerebrosidase which is located in the lysosomes. When the enzyme is defective, glucocerebroside accumulates and can collect in the spleen, live, kidneys, lungs, brain and bone marrow. Macrophages are unable to eliminate the accumulated enzyme, which accumulates in fibrils, and turn into Gaucher cells. The glucocerebrosidase deficiency in Gaucher patients promotes widespread accumulation of substrate glycosphingolipids in various organs, including the brain.

Neurological symptoms occur in some types of Gaucher disease. Neurological symptoms in Type I Gaucher disease include impaired olfaction and cognition. Neurological symptoms in Type II Gaucher disease include serious convulsions, hypertonia, mental retardation and apnea. Neurological symptoms in Type III Gaucher disease include muscle twitches known as myoclonus, convulsions, dementia and ocular muscle apraxia. Parkinson's disease is more common in Gaucher disease patients and their heterozygous carrier relatives than in the general population. Gaucher disease types II and III exhibit neurological defects which cannot be treated with protein replacement since the protein injected into the blood stream cannot penetrate the blood-brain barrier. Gene therapy (such as with an intravascular injection) may be advantageous when capable of transferring a nucleotide of interest to wide areas of the brain to treat the widespread pathology of the neurological disorder.

In some embodiments, the subject has one or more neurodegenerative diseases or lysosome storage diseases. In some embodiments, the subject has one or more neurodegenerative disease or lysosome storage diseases described herein.

Suitably, the subject may have Parkinson's disease and dementia. In some embodiments, the subject may have Parkinson's disease and Parkinson's dementia. In some embodiments, the subject may have Parkinson's disease and dementia with Lewy bodies.

In some embodiments, the subject may have Gaucher disease and Parkinson's disease. In some embodiments, the subject may have Gaucher disease and dementia.

The skilled person will understand that they can combine all features of the invention disclosed herein without departing from the scope of the invention as disclosed.

Preferred features and embodiments of the invention will now be described by way of nonlimiting examples.

The practice of the present invention will employ, unless otherwise indicated, conventional techniques of chemistry, biochemistry, molecular biology, microbiology and immunology, which are within the capabilities of a person of ordinary skill in the art. Such techniques are explained in the literature. See, for example, Sambrook, J., Fritsch, E.F. and Maniatis, T. (1989) Molecular Cloning: A Laboratory Manual, 2nd Edition, Cold Spring Harbor Laboratory Press; Ausubel, F.M. et al. (1995 and periodic supplements) Current Protocols in Molecular Biology, Ch. 9, 13 and 16, John Wiley & Sons; Roe, B., Crabtree, J. and Kahn, A. (1996) DNA Isolation and Sequencing: Essential Techniques, John Wiley & Sons; Polak, J.M. and McGee, J.O'D. (1990) In Situ Hybridization: Principles and Practice, Oxford University Press; Gait, M.J. (1984) Oligonucleotide Synthesis: A Practical Approach, IRL Press; and Lilley, D.M. and Dahlberg, J.E. (1992) Methods in Enzymology: DNA Structures Part A: Synthesis and Physical Analysis of DNA, Academic Press. Each of these general texts is herein incorporated by reference.

### EXAMPLES

### EXAMPLE 1

### Materials and Methods

### Virus production and purification

Viral library: Recombinant AAV vectors were produced by transfection of HEK293T cells. Plasmids, transfected using Polyfect transfection reagent (Qiagen) were: pXX6 (helper plasmid) and the library plasmidic preparation (at low concentration). Three days after cells were lysed in hypertonic buffer and the supernatant was concentrated (8% PEG8000). A discontinuous iodixanol gradient was prepared to isolate the viral particles. Upon ultracentrifugation, the purified library particles were aspirated from the layer containing 40% iodixanol and dialyzed against phosphate buffer solution (PBS). The virus titer was determined using AAVpro^{©} Titration Kit Ver2 (TaKaRa).

AAV-Se viral preparation: AAVs were produced as above with some modification in the transfection procedure that was performed by polyethylenimine (PEI) (Polyscience) co-transfection of three different plasmids: transgene-containing plasmid (pAAV-CBA-ZsGreen), packaging plasmid engineered from AAV9 plasmid to present in the capsid sequence the newly discovered peptides between amino acid positions 588 and 589 of the wild type AAV9 VP1 capsid protein, and pHelper (Agilent).

### AAV vector injection in mice and tissue collection

Viral preparations were injected in the mice tail with different viral concentrations (from 1*10¹⁰ for the library injection and 1*10¹² vg/mL for AAV-Se/AAV9 preparations) in a total volume of 100 µl. Three weeks after injection, all mice were sacrificed, and tissues harvested. Briefly, mice were anesthetized and perfused with PBS. Upon this treatment brain, the cortex and other off-target organs (liver, spleen, lung, kidney, heart and the quadricep muscle) were collected. Brain hemispheres were separated: one half was post-fixed in 4% PFA for immunofluorescence analysis, the other further sectioned, quick frozen on dry-ice for DNA extraction. Off-target tissues were collected similarly.

### AAV vector injection in marmosets and tissue collection

In collaboration with Dr. Ingrid Philippens at the Biomedical Primate Research Centre (BPRC, Nederland) 4 marmosets (*Callithrix jacchus*) were injected. Briefly, 1 ml of viral preparation was injected (viral dose: 1×10(13)vg per animal for AAV-Se2 and AAV9 preparations). Three weeks after injection, marmosets were sacrificed, and tissues harvested. Various brain areas were collected: the cortex, cerebellum, striatum, hippocampus, as well as off-target organs (liver, spleen, lung, kidney, heart and muscles). All the specimens were split in two, so that one half was post-fixed in 4% PFA for immunofluorescence analysis, and the other quick frozen for DNA extraction, and sent to our laboratory for further analysis.

### Library Processing and Analysis

DNA was purified from selected areas (QIAmp DNA Blood Mini Kit, Qiagen) and PCR-amplified for two different purposes: generation of a new library (Korbelin and Treppel (2017) Hum Gene Ther Methods) and analysis by New Generation Sequencing (NGS) of specimen (Amplicon EZ, Genewiz). Data analysis was performed by a custom script. Collected tissues (cortex and all the off-target tissues, see paragraph: "AAV vector injection in mice and tissue collection").

### Molecular characterization of AAV-Se in animal tissues

Total DNA was isolated from animal tissues (QIAmp DNA Blood Mini Kit, Qiagen) and the quantification of vector transgene expression was calculated by qRT-PCR relative to the ZsGreen transgene. The DNA levels were normalized against an amplicon of either the mouse Lmnb2, or the marmoset LMNB2 gene amplified from genomic DNA. Tissues, embedded in dry ice, were sectioned using a cryostat. Free-floating 50 µm-thick coronal sections were incubated overnight (4°C) with the primary antibody and the day after washed and incubated with the secondary antibody and DAPI. Sections were mounted (Dako) and confocal images were captured TCS SP5 Laser Scanning Confocal microscope (Leica Microsystems Ltd). Primary antibody: rabbit anti-ZsGreen (1:500; Clontech), mouse anti-NeuN (1:300; Merck Millipore), chicken anti-GFAP (1:500; Abcam), anti-chicken-MAP2 (1:500, Abcam), anti-rat CD31 (1:500, BD bioscience).

### Results

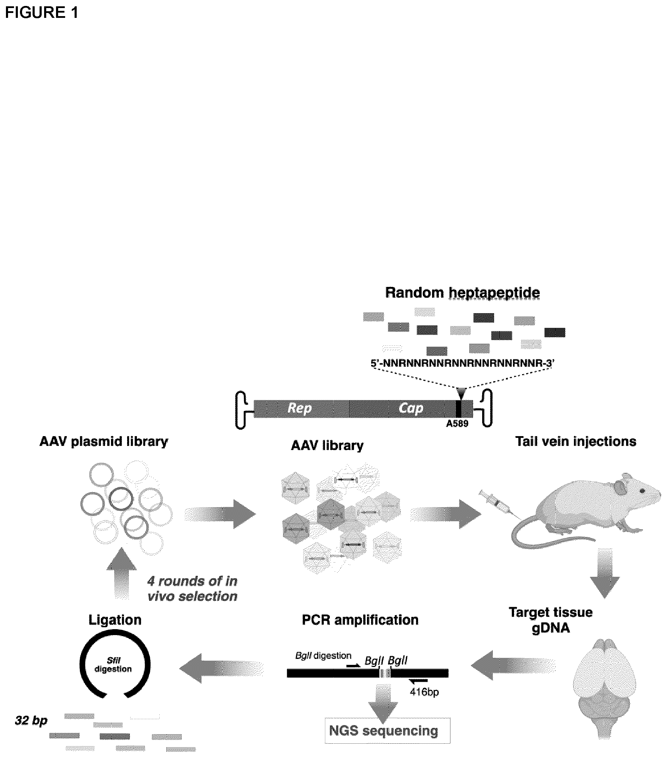

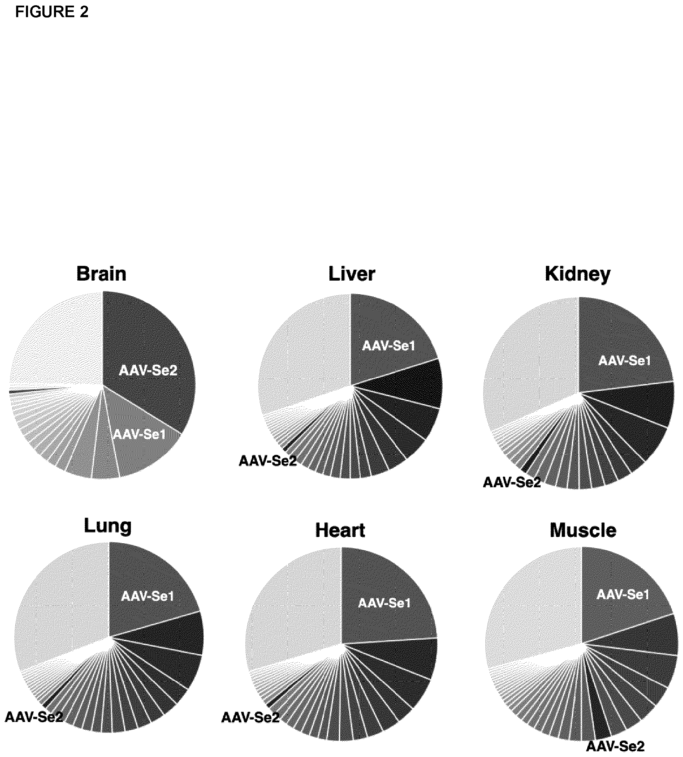

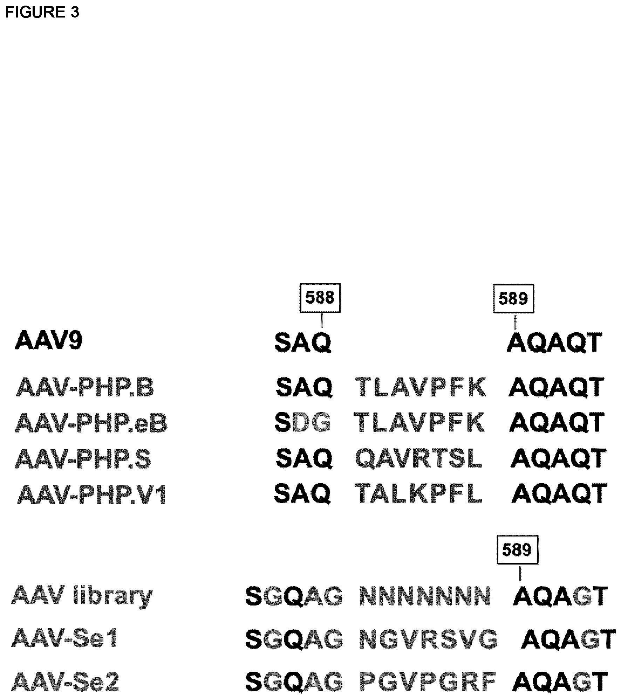

We performed an in vivo directed evolution screening approach using an AAV9 display peptide library to select novel brain targeting capsid variants. A peptide library including 7 random amino acids (7-AA) was inserted between amino acids 588 and 589 (numbering related to the VP1 protein) of the AAV9 capsid. Deep sequencing indicated the presence of 1×10⁸ different sequences in the AAV plasmid library. In order to prevent LY6A binding (AAV-PHP.eB receptor), the selection was carried out using BALB/c mice, that present inactivating mutations for this locus. We carried out four consecutive rounds of capsid selection by injecting the AAV library in the tail vein of an adult mice and retrieving the viral DNA from the cerebral cortex 3 weeks after the injections (Figure 1). After next-generation sequencing of the viral DNA in brain cells in each round of infection, we identified two capsid variants that were strongly enriched at the final round 4 that were named AAV-Se1 and AAV-Se2 (Figure 2). The exact sequence of the peptides inserted after position 588 for the new engineered capsids AAV-Se1 and AAV-Se2 are reported in Figure 3.

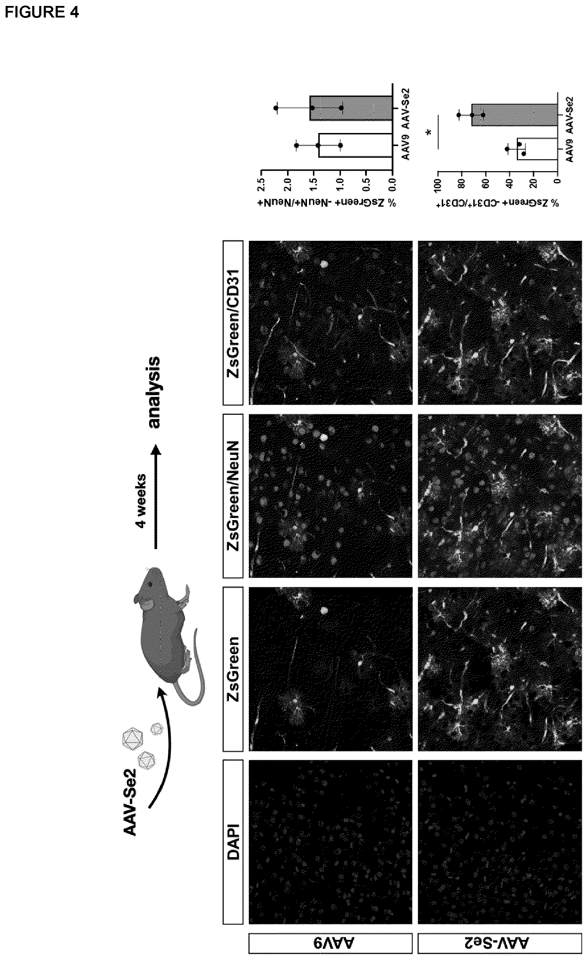

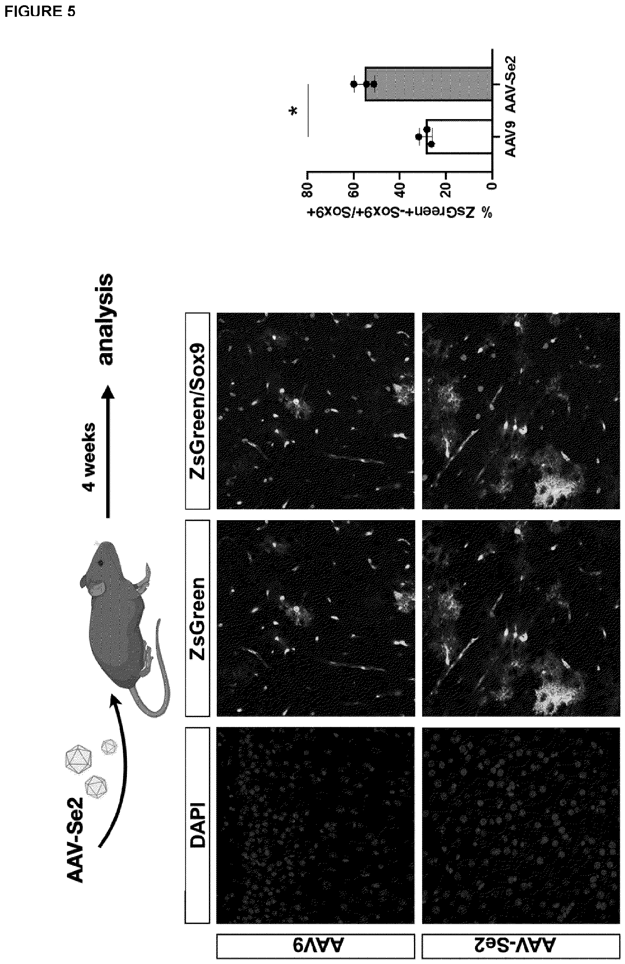

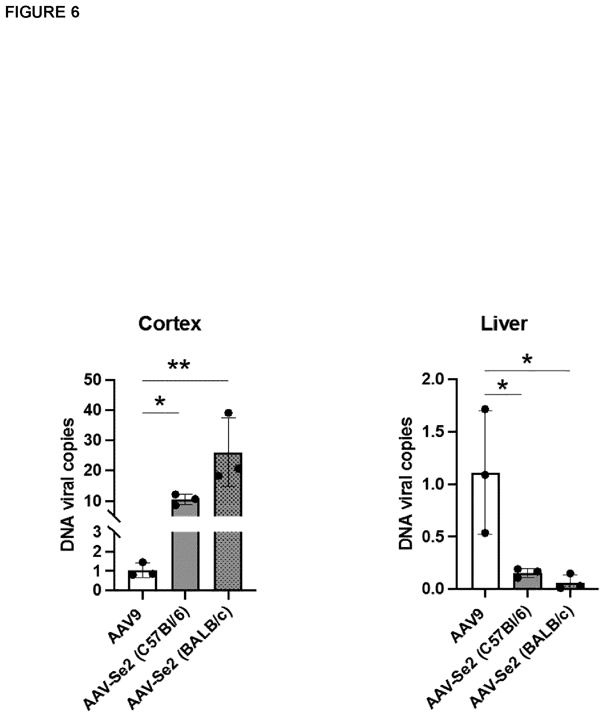

Next, we packaged a vector expressing Zsgreen under the control of the constitutive CBA promoter to compare the tropism efficiency and biodistribution of the two engineered AAVs compared with the standard and unmodified AAV9 capsid. Intravascular delivery in adult mice was carried out by tail vein injections with 1 × 10(11) vector genomes (vg)/mouse for each AAV capsid and 3 weeks later distribution of ZsGreen+ cells was visualized by double immunofluorescence with markers for neurons (NeuN), endothelial cells (CD31) and astrocytes (Sox9) (Figure 4). This analysis showed that AAV-Se2 has significant higher transduction efficiency in endothelial cells (about 2-fold increase) and astrocytes (about 3-fold increase) respect to the AAV9 capsid (Figures 4,5). On contrary, the transduced NeuN+ neuronal cell fraction was comparable between AAV-Se2 and AAV9 (Figure 4). A critical issue about intravascular delivery is the transduction of peripheral organs and in particular of the liver which might lead to hepatocyte sufferance and metabolic alterations. Interestingly, AAV-Se2 showed a strongly reduced tropism for the liver with about 10-fold reduction of viral DNA copies compared to AAV9 (Figure 6). General transduction pattern of AAV-Se2 was comparable in C57BI/6 (Ly6a active) and BALB/c (Ly6a deficient) mice indicating that its transduction efficiency does not depend by the presence of a Ly6a functional receptor (Figure 6).

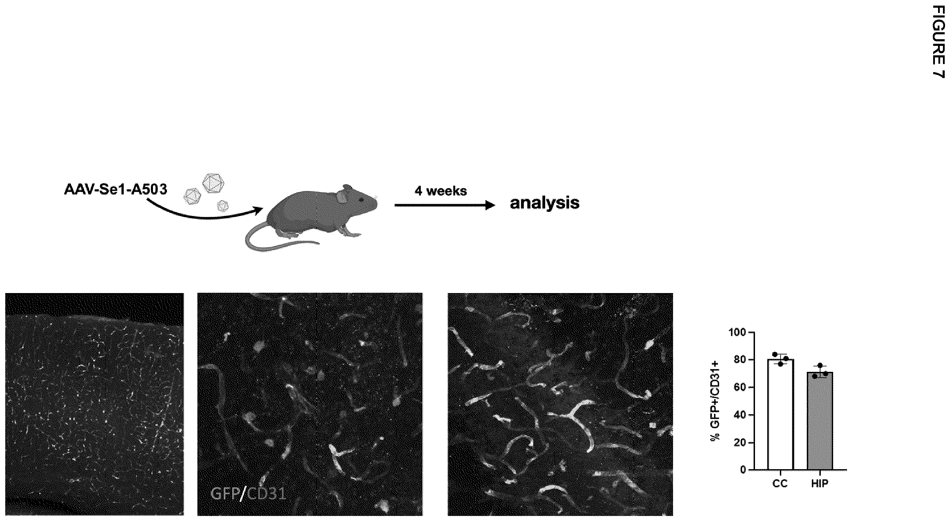

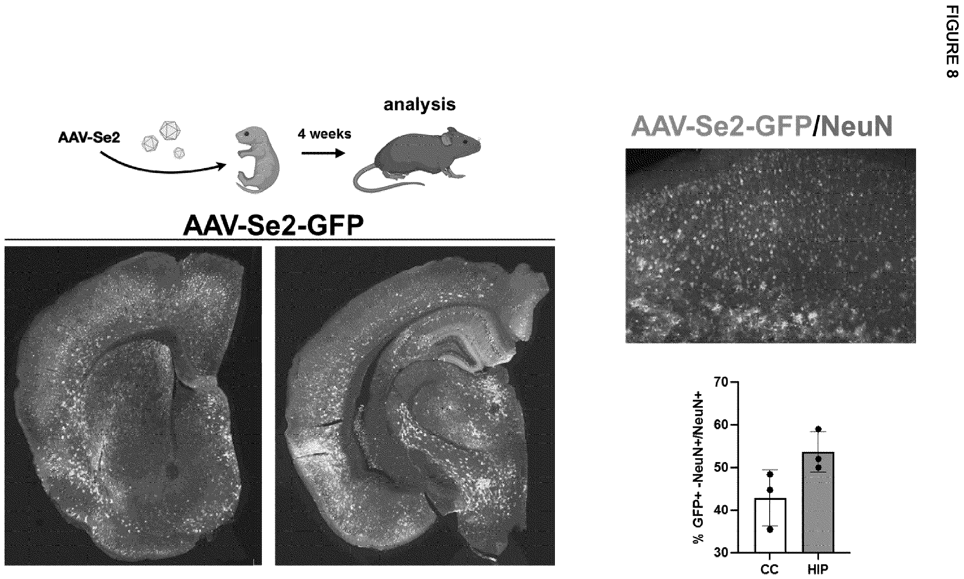

Next, we tested the AAV-Se1 capsid in which we introduced an additional mutation (R503A) in order to inactivate the binding to galactose of the AAV9 capsid. Interestingly, the resulting AAV-Se1-A503 capsid through the intravascular route transduced selectively the endothelium with a total efficiency of about 80% of total CD31+ endothelial cells throughout the brain (Figure 7). Next, we tested the transduction pattern of AAV-Se2 after intravascular delivery in P1-3 mouse pups through facial vein injections with 3 × 10(11) vg/animal (Figure 8). Three weeks after viral delivery, brains were isolated and processed by immunofluorescence for GFP and the neuronal marker NeuN (Figure 8). This analysis showed a much higher fraction of brain neurons reaching up to 50% of total NeuN+ cells transduced with AAV-Se2 (Figure 8). These results provide evidence that AAV-Se2 transduction in newborn animals transduces a higher fraction of brain neurons respect to the delivery in adults. To determine the translational potential of AAV-Se2 for treating human neuronal pathologies, we sought to assess the brain transduction potential in non-human primates and, in particular, in adult Marmosets (*Callithrix jacchus*).

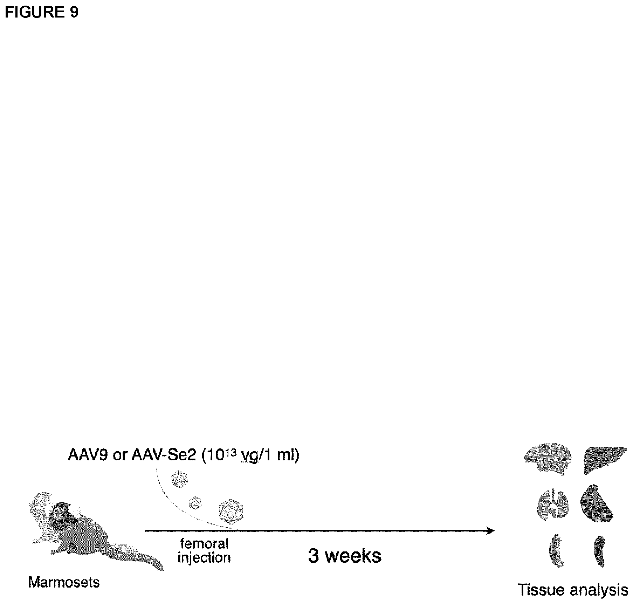

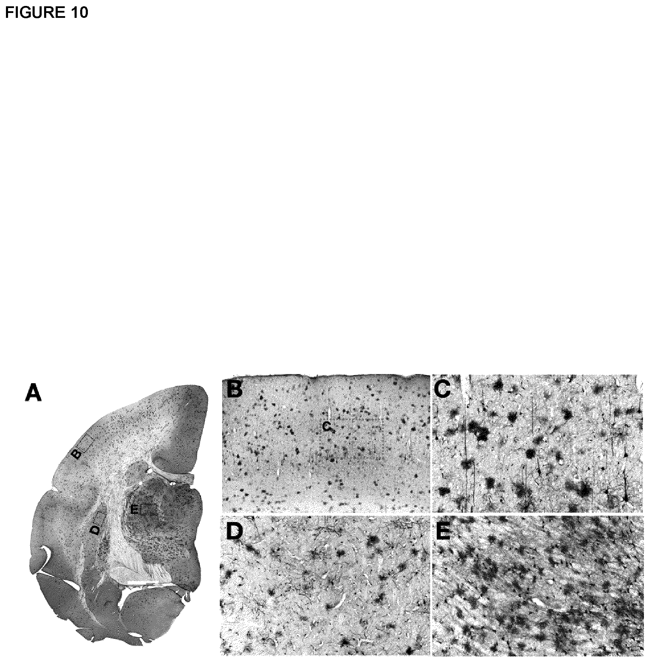

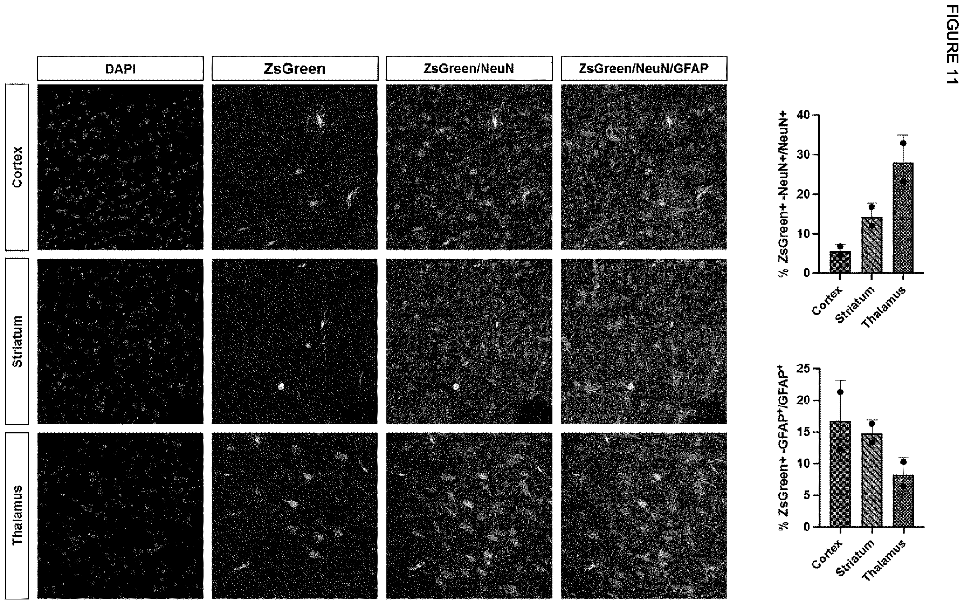

Thus, two adult Marmosets were injected through the femoral vein with either AAV-Se2 or standard AAV9 at the dose of 1 × 10(13) vg/animal expressing the ZsGreen reporter (Figure 9). Injected animals recovered well from the surgery and did not show any detectable alterations in behavior or liver metabolic enzymes during the three weeks before the sacrifice (Figure 9). Then, the cerebral autoptic tissue was collected and subjected to immunostaining for the viral reporter ZsGreen and the neuronal (NeuN) and astrocyte (GFAP) specific markers. ZsGreen immunohistochemistry in AAV-Se2 transduced brain tissues showed a widespread expression of the viral reporter in cerebral cortex, caudate nucleus, thalamus and cerebellum (Figure 10). Transduced neuronal and astrocytic cell fractions were quantified by double immunostaining, showing that 6 ± 2%, 15 ± 3%, 28 ± 6% of NeuN+ neurons and 17 ± 7%, 14 ± 2%, 8 ± 2% of GFAP+ astrocytes were infected in cerebral cortex, caudate nucleus and thalamus, respectively (Figure 11).

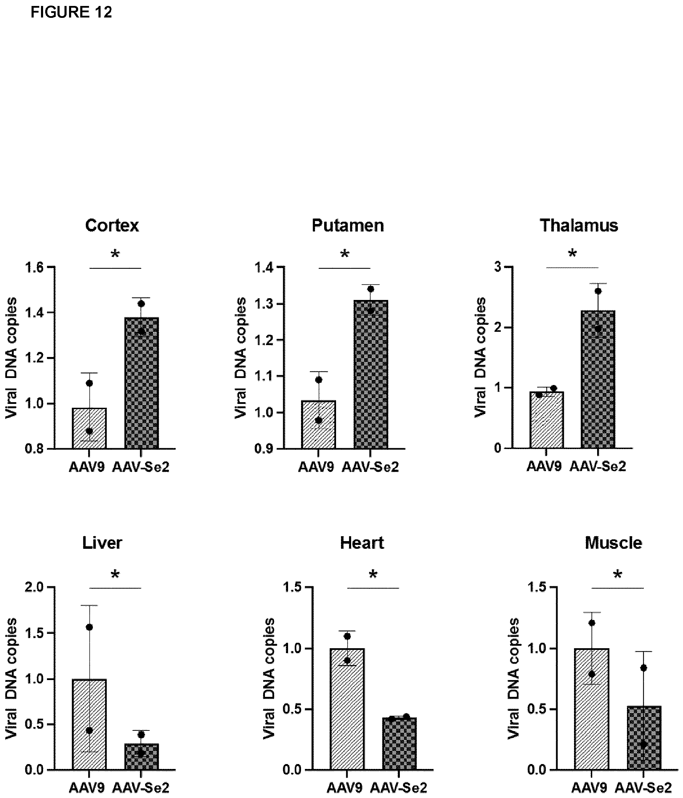

Finally, we ascertained the transduction levels of AAV-Se2 and AAV9 by quantifying the viral DNA copy number by qPCR analysis (Figure 12). In brain tissue lysates, we measured a general 20% to 50% increase in viral DNA copy of AAV-Se2 respect to standard AAV9 (Figure 12). Conversely, AAV-Se2 viral DNA was reduced more than 50% in peripheral organs such as liver, heart and muscles (Figure 12). In overall, AAV-Se2 showed a significant improved efficiency in brain transduction upon systemic delivery by the intravascular route with a marked reduction in targeting of peripheral organs with respect to the standard AAV9 capsid.

All publications mentioned in the above specification are herein incorporated by reference. Various modifications and variations of the disclosed products, compositions, uses and methods of the invention will be apparent to the skilled person without departing from the scope and spirit of the invention. Although the invention has been disclosed in connection with specific preferred embodiments, it should be understood that the invention as claimed should not be unduly limited to such specific embodiments. Indeed, various modifications of the disclosed modes for carrying out the invention, which are obvious to the skilled person are intended to be within the scope of the following claims.

### EMBODIMENTS

Various features and embodiments of the present invention will now be described with reference to the following numbered paragraphs:
1. An adeno-associated viral (AAV) capsid protein, wherein the capsid protein comprises:
   (a) a peptide comprising or consisting of the amino acid sequence PGVPGRF (SEQ ID NO: 1), or a variant thereof having up to two amino acid substitutions, additions or deletions; or
   (b) a peptide comprising or consisting of the amino acid sequence NGVRSVG (SEQ ID NO: 3), or a variant thereof having up to two amino acid substitutions, additions or deletions.
2. The AAV capsid protein of paragraph 1, wherein:
   (a) the peptide comprises or consists of the amino acid sequence AGPGVPGRF (SEQ ID NO: 2), or a variant thereof having up to two amino acid substitutions, additions or deletions; or
   (b) the peptide comprises or consists of the amino acid sequence AGNGVRSVG (SEQ ID NO: 4), or a variant thereof having up to two amino acid substitutions, additions or deletions.
3. The AAV capsid protein of paragraph 1 or 2, wherein the AAV capsid protein is an AAV VP1 capsid protein.
4. The AAV capsid protein of any preceding paragraph, wherein the AAV is AAV9.
5. The AAV capsid protein of any preceding paragraph, wherein the peptide is not at the N- or C-terminus of the AAV capsid protein.
6. The AAV capsid protein of any preceding paragraph, wherein the peptide is positioned immediately after a position corresponding to amino acid 588 of SEQ ID NO: 5.
7. The AAV capsid protein of any preceding paragraph, wherein:
   (a) the capsid protein comprises or consists of an amino acid sequence that has at least 75% sequence identity to SEQ ID NO: 6 or 7; or
   (b) the capsid protein comprises or consists of an amino acid sequence that has at least 75% sequence identity to SEQ ID NO: 8 or 9.
8. The AAV capsid protein of any preceding paragraph, wherein the capsid protein further comprises the mutation A587G and/or Q592A, wherein the amino acids are numbered with reference to SEQ ID NO: 5.
9. The AAV capsid protein of any preceding paragraph, wherein the capsid protein further comprises the mutation W503A, wherein the amino acids are numbered with reference to SEQ ID NO: 5.
10. The AAV capsid protein of any preceding paragraph, wherein the capsid protein further comprises the mutation K449R, wherein the amino acids are numbered with reference to SEQ ID NO: 5.
11. The AAV capsid protein of any preceding paragraph, wherein:
   (a) the capsid protein comprises or consists of an amino acid sequence that has at least 75% sequence identity to SEQ ID NO: 10; or
   (b) the capsid protein comprises or consists of an amino acid sequence that has at least 75% sequence identity to SEQ ID NO: 11.
12. An adeno-associated viral (AAV) particle comprising the AAV capsid protein of any preceding paragraph.
13. The AAV particle of paragraph 12, wherein the AAV particle comprises a polynucleotide comprising a nucleotide sequence of interest.
14. The AAV particle of paragraph 12 or 13, wherein the polynucleotide further comprises one or more AAV ITR.
15. The AAV particle of paragraph 14, wherein the one or more AAV ITR is one or more AAV2 ITR.
16. The AAV particle of any one of paragraphs 13-15, wherein the nucleotide sequence of interest is operably linked to a promoter.
17. The AAV particle of any one of paragraphs 13-16, wherein the nucleotide sequence of interest is operably linked to an enhancer.
18. A pharmaceutical composition comprising the adeno-associated viral (AAV) particle of any one of paragraphs 12-17, and a pharmaceutically acceptable carrier, diluent or excipient.
19. The adeno-associated viral (AAV) particle of any one of paragraphs 12-17 for use in therapy.
20. Use of the adeno-associated viral (AAV) particle of any one of paragraphs 12-17 for the manufacture of a medicament.
21. A method of treating or preventing a disease, the method comprising a step of administering the adeno-associated viral (AAV) particle of any one of paragraphs 12-17 to a subject in need thereof.
22. The adeno-associated viral (AAV) particle of any one of paragraphs 12-17 for use in treating or preventing a disease of the brain, or central and/or peripheral nervous system.
23. Use of the adeno-associated viral (AAV) particle of any one of paragraphs 12-17 for the manufacture of a medicament for treating or preventing a disease of the brain, or central and/or peripheral nervous system.
24. A method of treating or preventing a disease of the brain, or central and/or peripheral nervous system, the method comprising a step of administering the adeno-associated viral (AAV) particle of any one of paragraphs 12-17 to a subject in need thereof.
25. The AAV particle for use according to, the use of or the method of any one of paragraphs 22-24, wherein the disease is a neurodegenerative disease.
26. The AAV particle for use according to, the use of or the method of any one of paragraphs 22-25, wherein the disease is dementia.
27. The AAV particle for use according to, the use of or the method of any one of paragraphs 22-26, wherein the disease is selected from the group consisting of Parkinson's disease, Parkinson's dementia, Lewy Body dementia, diffuse Lewy body dementia, Lewy body variant of Alzheimer's disease, multiple system atrophy and Gaucher disease.
28. The AAV particle for use according to, the use of or the method of any one of paragraphs 19-27, wherein the AAV particle is administered systemically or locally in the brain.
29. The AAV particle for use according to, the use of or the method of any one of paragraphs 19-28, wherein the AAV particle is administered intravenously or intrathecally.
30. The AAV particle for use according to, the use of or the method of any one of paragraphs 19-29, wherein the subject is a juvenile.
31. The AAV particle for use according to, the use of or the method of any one of paragraphs 19-30, wherein the subject is a paediatric subject, optionally wherein the subject is a neonatal subject or an infantile subject.
32. Use of a peptide for targeting a viral or non-viral particle to cells of the brain, or central and/or peripheral nervous system, wherein the peptide:
   (a) comprises or consists of the amino acid sequence PGVPGRF (SEQ ID NO: 1), or a variant thereof having up to two amino acid substitutions, additions or deletions; or
   (b) comprises or consists of the amino acid sequence NGVRSVG (SEQ ID NO: 3), or a variant thereof having up to two amino acid substitutions, additions or deletions.
33. Use of a peptide for targeting a viral or non-viral particle to cells of the brain, or central and/or peripheral nervous system, wherein the peptide:
   (a) comprises or consists of the amino acid sequence AGPGVPGRF (SEQ ID NO: 2), or a variant thereof having up to two amino acid substitutions, additions or deletions; or
   (b) comprises or consists of the amino acid sequence AGNGVRSVG (SEQ ID NO: 4), or a variant thereof having up to two amino acid substitutions, additions or deletions.
34. Use of an adeno-associated viral (AAV) capsid protein of any one of paragraphs 1-11 for targeting a viral particle to cells of the brain, or central and/or peripheral nervous system.
35. Use of a peptide for increasing the efficiency of transduction of cells of the brain by a viral particle, wherein the peptide:
   (a) comprises or consists of the amino acid sequence PGVPGRF (SEQ ID NO: 1), or a variant thereof having up to two amino acid substitutions, additions or deletions; or
   (b) comprises or consists of the amino acid sequence NGVRSVG (SEQ ID NO: 3), or a variant thereof having up to two amino acid substitutions, additions or deletions.
36. Use of a peptide for increasing the efficiency of transduction of cells of the brain by a viral particle, wherein the peptide:
   (a) comprises or consists of the amino acid sequence AGPGVPGRF (SEQ ID NO: 2), or a variant thereof having up to two amino acid substitutions, additions or deletions; or
   (b) comprises or consists of the amino acid sequence AGNGVRSVG (SEQ ID NO: 4), or a variant thereof having up to two amino acid substitutions, additions or deletions.
37. Use of an adeno-associated viral (AAV) capsid protein of any one of paragraphs 1-11 for increasing the efficiency of transduction of cells of the brain by a viral particle.
38. Use of a peptide for decreasing tropism of a viral or non-viral particle for non-brain tissue, wherein the peptide:
   (a) comprises or consists of the amino acid sequence PGVPGRF (SEQ ID NO: 1), or a variant thereof having up to two amino acid substitutions, additions or deletions; or
   (b) comprises or consists of the amino acid sequence NGVRSVG (SEQ ID NO: 3), or a variant thereof having up to two amino acid substitutions, additions or deletions.
39. Use of a peptide for decreasing tropism of a viral or non-viral particle for non-brain tissue, wherein the peptide:
   (a) comprises or consists of the amino acid sequence AGPGVPGRF (SEQ ID NO: 2), or a variant thereof having up to two amino acid substitutions, additions or deletions; or
   (b) comprises or consists of the amino acid sequence AGNGVRSVG (SEQ ID NO: 4), or a variant thereof having up to two amino acid substitutions, additions or deletions.
40. Use of an adeno-associated viral (AAV) capsid protein of any one of paragraphs 1-11 for decreasing tropism of a viral particle for non-brain tissue.
41. The use of any one of paragraphs 32-40, wherein the viral particle is an AAV particle.
42. The use of any one of paragraphs 32-34 or 38-40, wherein the non-viral particle is a nanoparticle, optionally a lipid nanoparticle.
43. The use of any one of paragraphs 32-37, 41 or 42, wherein the cells are cells of the cerebral cortex, caudate nucleus, thalamus and/or cerebellum.
44. The use of any one of paragraphs 32-37, 41 or 42, wherein the cells are brain endothelial cells, brain astrocytes or brain neuronal cells.
45. The use of any one of paragraphs 38-40, 41 or 42, wherein the non-brain tissue is liver, spleen, lung, kidney, heart and/or muscle.

## Claims

1. An adeno-associated viral (AAV) capsid protein, wherein the capsid protein comprises:
(a) a peptide comprising or consisting of the amino acid sequence PGVPGRF (SEQ ID NO: 1), or a variant thereof having up to two amino acid substitutions, additions or deletions; or
(b) a peptide comprising or consisting of the amino acid sequence NGVRSVG (SEQ ID NO: 3), or a variant thereof having up to two amino acid substitutions, additions or deletions.

2. The AAV capsid protein of claim 1, wherein:
(a) the peptide comprises or consists of the amino acid sequence AGPGVPGRF (SEQ ID NO: 2), or a variant thereof having up to two amino acid substitutions, additions or deletions; or
(b) the peptide comprises or consists of the amino acid sequence AGNGVRSVG (SEQ ID NO: 4), or a variant thereof having up to two amino acid substitutions, additions or deletions.

3. The AAV capsid protein of claim 1 or 2, wherein the AAV capsid protein is an AAV VP1 capsid protein.

4. The AAV capsid protein of any preceding claim, wherein the AAV is AAV9.

5. The AAV capsid protein of any preceding claim, wherein the peptide is positioned immediately after a position corresponding to amino acid 588 of SEQ ID NO: 5.

6. The AAV capsid protein of any preceding claim, wherein:
(a) the capsid protein comprises or consists of an amino acid sequence that has at least 75% sequence identity to SEQ ID NO: 6 or 7; or
(b) the capsid protein comprises or consists of an amino acid sequence that has at least 75% sequence identity to SEQ ID NO: 8 or 9.

7. The AAV capsid protein of any preceding claim, wherein the capsid protein further comprises
(a) the mutation A587G and/or Q592A, wherein the amino acids are numbered with reference to SEQ ID NO: 5;
(b) the mutation W503A, wherein the amino acids are numbered with reference to SEQ ID NO: 5; and/or
(c) the mutation K449R, wherein the amino acids are numbered with reference to SEQ ID NO: 5.

8. The AAV capsid protein of any preceding claim, wherein:
(a) the capsid protein comprises or consists of an amino acid sequence that has at least 75% sequence identity to SEQ ID NO: 10; or
(b) the capsid protein comprises or consists of an amino acid sequence that has at least 75% sequence identity to SEQ ID NO: 11.

9. An adeno-associated viral (AAV) particle comprising the AAV capsid protein of any preceding claim.

10. The adeno-associated viral (AAV) particle of claim 9 for use in therapy.

11. The adeno-associated viral (AAV) particle of claim 9 for use in treating or preventing a disease of the brain, or central and/or peripheral nervous system.

12. The AAV particle for use according to claim 10 or 11, wherein the AAV particle is administered systemically or locally in the brain.

13. Use of a peptide for targeting a viral or non-viral particle to cells of the brain, or central and/or peripheral nervous system, wherein the peptide:
(a) comprises or consists of the amino acid sequence PGVPGRF (SEQ ID NO: 1), or a variant thereof having up to two amino acid substitutions, additions or deletions; or
(b) comprises or consists of the amino acid sequence NGVRSVG (SEQ ID NO: 3), or a variant thereof having up to two amino acid substitutions, additions or deletions.

14. Use of a peptide for increasing the efficiency of transduction of cells of the brain by a viral particle, wherein the peptide:
(a) comprises or consists of the amino acid sequence PGVPGRF (SEQ ID NO: 1), or a variant thereof having up to two amino acid substitutions, additions or deletions; or
(b) comprises or consists of the amino acid sequence NGVRSVG (SEQ ID NO: 3), or a variant thereof having up to two amino acid substitutions, additions or deletions.

15. Use of a peptide for decreasing tropism of a viral or non-viral particle for non-brain tissue, wherein the peptide:
(a) comprises or consists of the amino acid sequence PGVPGRF (SEQ ID NO: 1), or a variant thereof having up to two amino acid substitutions, additions or deletions; or
(b) comprises or consists of the amino acid sequence NGVRSVG (SEQ ID NO: 3), or a variant thereof having up to two amino acid substitutions, additions or deletions.
